# FASCICULE DE BREVET EUROPEEN

(11) **EP 1 542 807 B1**
(45) Date de publication et mention de la délivrance du brevet: **22.02.2006**
(21) Numéro de dépôt: 03776944.5
(22) Date de dépôt: 26.09.2003
(51) Int. Cl.: B05B 11/00

(54) **DISPOSITIF DE PULVERISATION OU D INJECTION PERMETTANT DE DEL IVRER AU MOINS DEUX DOSES DETERMINEES DE PRODUIT**
SPRÜH- ODER INJEKTIONSVORRICHTUNG ZUR DOSIERTEN ABGABE MINDESTENS ZWEIER DOSEN EINES PRODUKTS
SPRAY DEVICE OR INJECTION DEVICE ENABLING DELIVERY OF AT LEAST TWO PREDETERMINED PRODUCT DOSES

(30) Priorité: 27.09.2002 FR 0212002
(43) Date de publication de la demande: 22.06.2005
(73) Titulaire: Becton Dickinson France, 38800 Le Pont de Claix (FR)
(72) Inventeur: VEDRINE, Lionel, F-38400 St. Martin d'Hères (FR); PEROT, Frédéric, F-38000 Grenoble (FR); BARRELLE, Laurent, F-38250 Saint Nizier du Moucherotte (FR)
(74) Mandataire: Brédeville, Odile Marie
(86) Numéro de dépôt international: PCT/FR2003/002836
(87) Numéro de publication internationale: WO 2004/028703

(56) Documents cités:
- US-A- 4 820 287
- US-A- 5 951 526
- US-B1- 6 382 204
- US-B1- 6 382 465

## Description

La présente invention concerne un dispositif de pulvérisation ou d'injection d'un produit d'intérêt sous forme liquide, permettant de délivrer successivement au moins une première dose et une deuxième dose dudit produit. L'invention est notamment applicable à un pulvérisateur nasal permettant de délivrer deux doses, une pour chaque narine.

Il est connu de réaliser un pulvérisateur nasal basé sur une structure générale de seringue, comprenant des moyens de butée amovibles. Ces moyens de butée permettent, lorsqu'ils sont en place, de délimiter une portion de course de la tige de piston, pour la délivrance d'une première dose, et peuvent être retirés ou effacés pour libérer le reste de la course de la tige de piston, pour permettre la délivrance de la deuxième dose. Les brevets américains n° 5 601 077 et 5 951 526, au nom de la demanderesse, illustrent cette technique.

Il est également connu par le brevet américain n° 6 382 204, également au nom de la demanderesse, conforme avec le préambule de la revendiaction 1, de réaliser un dispositif comprenant un étui recevant le corps de seringue et un poussoir formant la tige de piston, l'étui comprenant des ergots et le poussoir comprenant des rainures dans lesquelles ces ergots peuvent coulisser. Chaque rainure comprend deux portions longitudinales décalées angulairement et une portion intermédiaire raccordant ces deux portions longitudinales, perpendiculaires à l'axe du poussoir. Chaque ergot peut coulisser dans une première portion longitudinale jusqu'à venir en butée contre le poussoir dans la zone de raccordement de cette première portion longitudinale à la portion intermédiaire, puis, par rotation axiale du poussoir, emprunter la portion intermédiaire jusqu'à venir en regard de la deuxième portion longitudinale et coulisser ensuite dans cette deuxième portion longitudinale. Lesdites portions longitudinales définissent ainsi deux portions de course successives du poussoir, déterminant la délivrance respectivement des première et deuxième doses du produit.

Les dispositifs existants ont pour inconvénient de ne pas être d'une manipulation très aisée, qu'il s'agisse d'effacer lesdits moyens de butée selon la première technique citée, ou d'opérer le pivotement relatif du poussoir et de l'étui selon la deuxième technique citée.

Ces dispositifs ont également pour inconvénient de ne pas empêcher des erreurs d'utilisation.

En outre, la conception des rainures et des ergots précités est relativement complexe à réaliser pour l'obtention d'une bonne fiabilité de fonctionnement.

La présente invention vise à remédier à l'ensemble de ces inconvénients, en fournissant un dispositif à la fois aisé à manipuler, réduisant au minimum les erreurs d'utilisation, et restant d'une structure relativement simple et peu onéreuse à fabriquer.

Le dispositif qu'elle concerne comprend, de manière connue en soi :
- un récipient contenant le produit à pulvériser ou à injecter,
- un piston placé dans ce récipient, et obturant ce dernier, ledit piston étant déplaçable axialement par rapport au récipient dans un sens de référence permettant la propulsion distale du produit hors du récipient,
- un étui agencé pour recevoir et y fixer axialement le récipient,
- un poussoir assemblé avec l'étui, déplaçable par rapport à ce dernier, agencé pour venir en butée axiale contre le récipient ou le piston, et déplacer ledit piston dans le sens de référence,
- des moyens de contrôle de la course de déplacement du poussoir par rapport à l'étui, agencés pour diviser cette dernière en une première portion de course et une deuxième portion de course, déterminant la délivrance respectivement des première et deuxième doses.

Selon une première modalité de l'invention :
- l'étui ou le poussoir comprend au moins une patte mobile radialement entre une première position radiale normale, ou non contrainte, dans laquelle la patte ne fait pas obstacle au déplacement du poussoir par rapport à l'étui, et une deuxième position radiale, fléchie, sous contrainte, dans laquelle la patte fait obstacle à ce déplacement, cette patte comprenant une zone d'appui et une zone de non appui ;
- le poussoir ou l'étui, respectivement, comprend au moins une saillie comprenant une rampe contre laquelle vient porter ladite zone d'appui de la patte au cours du déplacement du poussoir par rapport à l'étui, dans le sens permettant l'injection ou la pulvérisation du produit, ce qui amène ladite patte dans ladite deuxième position radiale, puis en regard de laquelle vient ladite zone de non appui de la patte, ce qui permet le retour de ladite patte dans ladite première position radiale ; le poussoir ou l'étui, respectivement, comprend en outre au moins une butée contre laquelle la patte vient en appui lorsqu'elle est amenée dans ladite deuxième position radiale par ladite saillie, cet appui se produisant juste avant que ladite saillie vienne en regard de ladite zone de non appui,
- le dispositif étant conçu de telle sorte que le relâchement de l'effort exercé sur le poussoir, qui permet de déplacer ce poussoir par rapport à l'étui, permette de libérer l'appui de la patte contre ladite butée, et donc permette le retour de ladite patte dans ladite première position radiale, ladite saillie venant alors en regard de ladite zone de non appui, ce retour de ladite patte dans ladite première position radiale permettant de libérer le déplacement du poussoir pour une portion de course suivante, en vue de la pulvérisation ou de l'injection d'une dose suivante du produit.

Conformément à la présente invention, l'étui et le poussoir sont agencés pour être déplaçables axialement l'un par rapport à l'autre, dans un sens de poussée, générant le déplacement du piston dans le sens de référence.

Conformément à la présente invention, de manière générale, les moyens de contrôle comprennent :
- au moins une patte disposée sur le poussoir ou l'étui, mobile radialement entre une première position radiale, non contrainte, dans laquelle ladite patte ne fait pas obstacle au déplacement axial du poussoir, et une deuxième position fléchie, sous contrainte, dans laquelle ladite patte arrête le déplacement axial du poussoir, ladite patte comportant à son extrémité libre une zone ou élément d'appui agencé pour contribuer à la fois à l'arrêt du déplacement axial du poussoir et au fléchissement de la patte sous l'effet du déplacement axial du poussoir
- au moins une rampe coopérant avec ladite patte d'une extrémité dite initiale à une extrémité dite finale, disposée respectivement sur l'étui ou le poussoir, contre laquelle porte la zone ou élément d'appui de ladite patte, dans le sens de la poussée dudit poussoir, ladite rampe étant agencée pour amener ladite patte de sa première position normale, à sa deuxième position fléchie,
- au moins une butée, coopérant avec la zone ou élément d'appui de la patte, disposée respectivement sur l'étui ou le poussoir, respectivement au-delà ou en deçà de l'extrémité finale de la rampe dans le sens de la poussée, contre laquelle la zone ou élément d'appui de la patte vient finalement en contact, dans sa deuxième position fléchie,
- au moins une zone ou ouverture de non appui, qui est, soit disposée sur la patte, en deçà ou delà de ladite zone ou élément d'appui, selon que ladite patte est disposée sur le poussoir ou l'étui, soit disposée sur l'étui, en deçà de la butée, lorsque la patte est disposée sur le poussoir et l'élément d'appui coopère avec une rampe en creux ménagée sur l'étui, la dite zone ou ouverture de non appui étant adaptée pour permettre le retour de ladite zone ou élément d'appui, à sa position normale, non contrainte, à partir de la positon arrêtée et fléchie de la patte, lorsque la poussée sur le poussoir est relâchée.
- la première portion de course du poussoir étant déterminée par le déplacement de ce dernier jusqu'à sa position d'arrêt axial, consécutif au contact de la zone ou élément d'appui avec la butée, et la deuxième portion de course par le déplacement du poussoir, au-delà de la position axiale dans laquelle la zone ou ouverture de non appui a accueilli la zone ou élément d'appui.

Ainsi, il suffit à l'utilisateur d'exercer un effort sur le poussoir, de manière à déplacer ce dernier par rapport à l'étui, pour libérer une première dose de produit, jusqu'à blocage du déplacement du poussoir résultant de la venue de la patte en appui contre ladite butée, puis de relâcher cet effort pour libérer ce blocage et permettre, par un nouvel effort sur le poussoir, de délivrer la deuxième dose ou dose suivante de produit

La manipulation du dispositif selon l'invention n'implique donc aucun retrait ou effacement de moyens de butée, ni rotation axiale du poussoir par rapport à l'étui, et est donc particulièrement aisée. Le fonctionnement du dispositif élimine pratiquement tout risque d'erreur d'utilisation, et la conception de ce dispositif est plus simple que celle d'un dispositif selon la technique antérieure.

Ladite zone de non appui de la patte peut notamment être formée par une fenêtre aménagée dans la patte en retrait de l'extrémité de cette patte, ladite zone d'appui étant formée par la zone de cette patte reliant cette extrémité de la patte et cette fenêtre.

De préférence, le dispositif comprend au moins deux pattes, deux saillies et deux zones de butée diamétralement opposées, ce qui fiabilise le fonctionnement du dispositif.

Avantageusement, l'étui et le poussoir comprennent des moyens permettant de former au moins un "point dur" devant être franchi en début de délivrance d'une dose. Ces moyens permettent de prévenir toute pulvérisation ou injection involontaire d'une dose, et donc de fiabiliser encore l'utilisation du dispositif. Ces moyens peuvent notamment être constitués par au moins un ergot faisant saillie latéralement d'une patte telle que précitée et par au moins un bossage aménagé en correspondance sur l'étui ou le poussoir.

Pour sa bonne compréhension, l'invention est à nouveau décrite ci-dessous en référence au dessin schématique annexé représentant, à titre d'exemples non limitatifs, quatre formes de réalisation possibles du dispositif qu'elle concerne.
La figure 1 en est une vue en perspective selon une première forme de réalisation ;
la figure 2 en est une vue similaire à la figure 1, en coupe axiale ;
la figure 3 est une vue en coupe partielle du poussoir qu'il comprend ;
la figure 4 est une vue en coupe axiale de l'étui qu'il comprend ;
les figures 5 à 8 en sont des vues en coupe axiale, du dispositif selon la première forme de réalisation, respectivement en début d'injection d'une première dose, en fin d'injection de cette première dose, en début d'injection de la deuxième dose et en fin d'injection de cette deuxième dose ;
les figures 9 et 10 en sont des vues en coupe axiale du dispositif selon l'invention, respectivement selon deux variantes de la première forme de réalisation ;
la figure 11 en est une vue en coupe axiale sur un secteur, d'un dispositif selon la deuxième forme de réalisation de l'invention ;
les figures 12 à 15 en sont des vues partielles, en demi-coupe axiale, respectivement en début d'injection d'une première dose, en fin d'injection de cette première dose, en début d'injection de la deuxième dose et en fin d'injection de cette deuxième dose, selon la deuxième forme de réalisation, et
la figure 16 est une vue d'une troisième variante de la première forme de réalisation de l'invention
- la figure 17 représente en vue agrandie un détail concernant la rampe appartenant à l'étui du dispositif selon la première forme de réalisation (figures 1 à 8) de l'invention
- la figure 18 est une vue de face d'un dispositif, selon une troisième forme de réalisation de la présente invention ;
- la figure 19 est une vue en perspective de l'étui du dispositif selon la figure 18 ;
la figure 20 est une vue en perspective du poussoir du dispositif selon la figure 18 ;
- la figure 21 est une vue agrandie, à plat, de la rampe appartenant à l'étui selon la figure 19 ;
- la figure 22 représente en perspective, avec coupe partielle, un dispositif selon une quatrième forme de réalisation de l'invention ;
- la figure 23 représente en perspective, de manière séparée, l'étui et le poussoir du dispositif selon la figure 22 ;
- la figure 24 représente une vue en coupe axiale du dispositif selon la figure 22 ;
- les figures 25 à 30 représentent en demi-coupe axiale, différentes étapes de fonctionnement du poussoir par rapport à l'étui, à savoir respectivement :
   + position initiale, avant délivrance de la première dose ; cf. figure 25
   + fléchissement de la patte, la languette demeurant en position normale ; cf. figure 26
   + fléchissement de la patte et de la languette ; cf. figure 27
   + échappement de la patte par rapport à la rampe, avec fléchissement de la languette ; cf. figure 28
   + poursuite du déplacement de la patte, la languette échappant à la rampe, pour délivrer la deuxième dose ; cf. figure 29
   + poursuite du déplacement de la patte et de la languette, au-delà de la rampe ; cf. figure 30
   + position intermédiaire, avant délivrance de la deuxième dose.

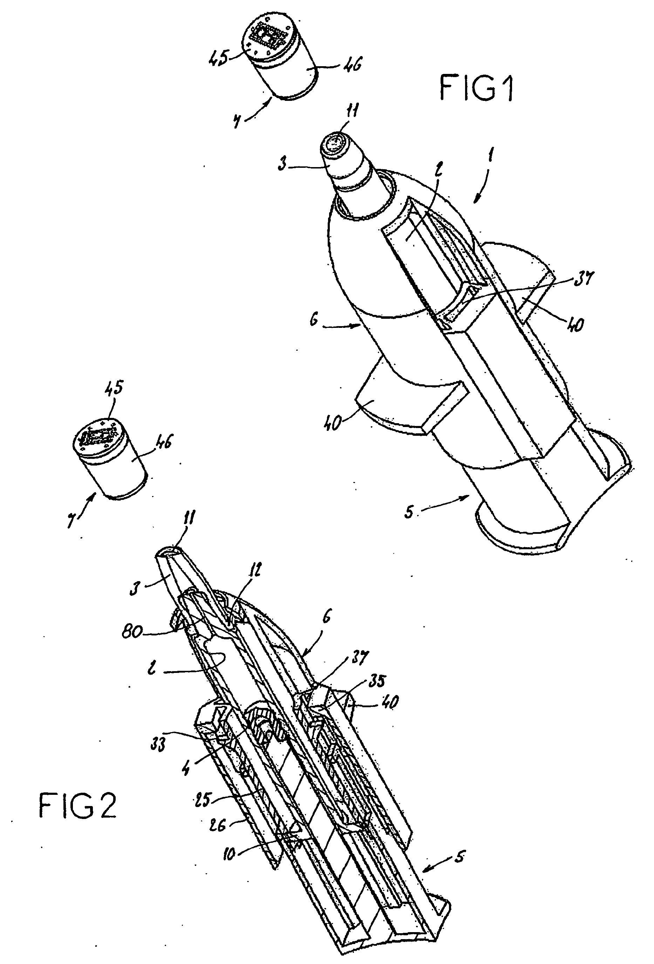

Les figures 1 à 8 représentent un dispositif 1 de pulvérisation nasale, selon une première forme de réalisation de l'invention, permettant de délivrer deux doses déterminées d'un produit d'intérêt sous forme liquide, une pour chaque narine.

Ce dispositif 1 comprend un corps de seringue 2, un embout de pulvérisation 3, un piston 4, un poussoir 5, un étui 6 et un capuchon de protection 7.

Le corps de seringue 2 est de type classique, par exemple en verre, avec une collerette proximale 10 et un conduit distal 80 d'écoulement.

L'embout 3 est emmanché sur l'extrémité distale du corps de seringue 2. Il forme une tête de pulvérisation 11 permettant de pulvériser le produit contenu dans le corps de seringue 2, et comprend une collerette proximale 12. Un tel embout 3 étant connu en lui-même, il n'est pas décrit plus en détails.

Le piston 4 est également de type classique, s'agissant d'un piston de seringue.

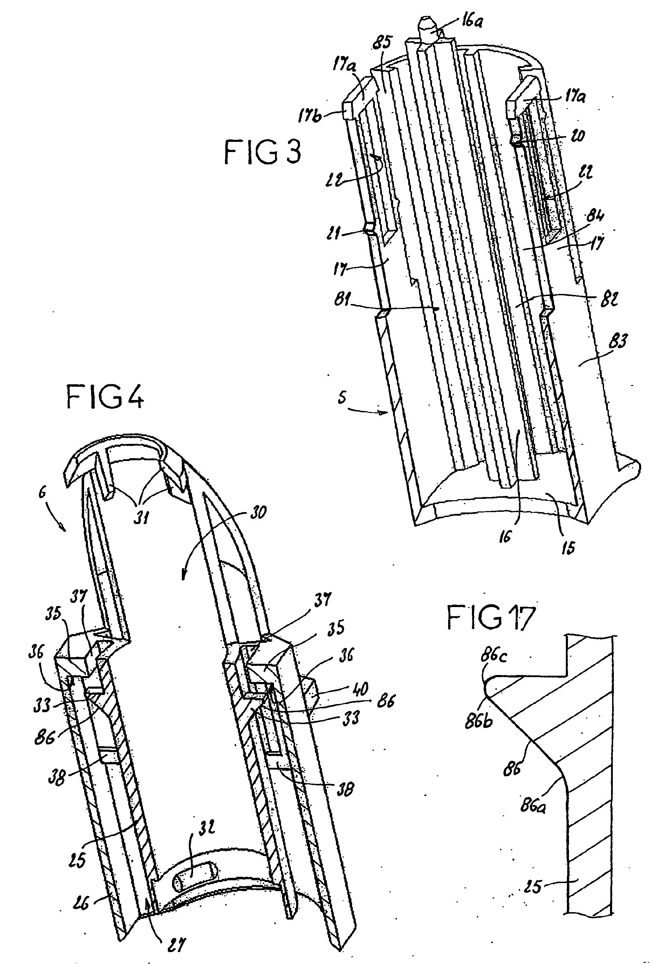

Comme le montrent plus particulièrement les figures 2 et 3, le poussoir 5 présente une forme générale cylindrique creuse, fermée par un fond proximal 15, et comprend une tige axiale 16 solidaire de ce fond 15, recevant le piston 4 à son extrémité distale grâce à un téton 16a qu'elle comprend. La paroi 83 du poussoir 5 est raidie par des nervures internes, longitudinales, 81 et 82, parallèles à la tige 16.

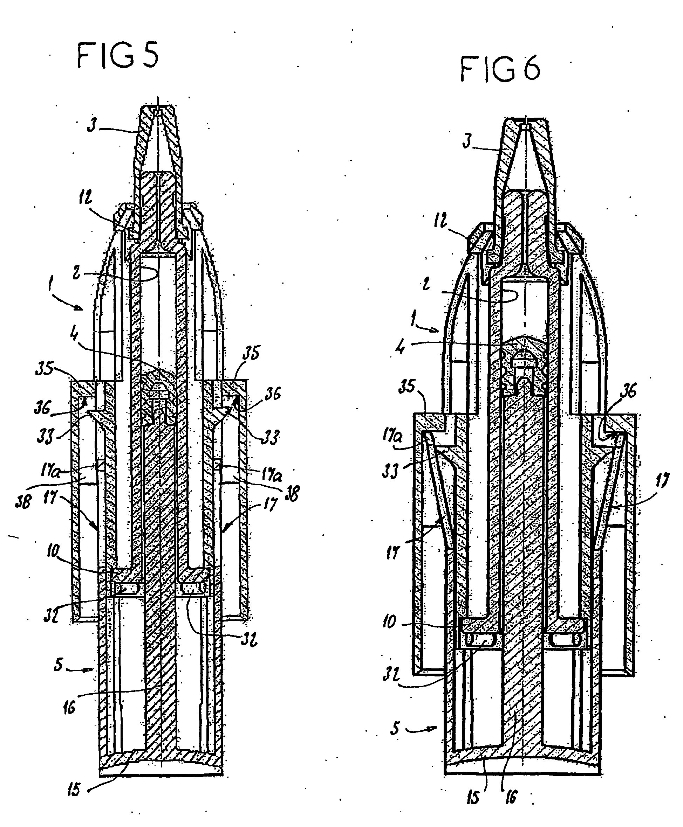

Le poussoir 5 comprend également, dans l'exemple représenté, quatre saignées 84, 85 aménagées longitudinalement à partir de son extrémité distale, délimitant deux pattes mobiles 17 diamétralement opposées. L'ensemble du poussoir 5 est réalisé en une matière plastique courante, légèrement flexible, la mobilité radiale ou tangentielle des pattes 17 résultant de la flexibilité de ce matériau. Cette flexibilité permet la mobilité des pattes 17 entre une première position normale radiale, non contrainte, interne, de non déformation, montrée sur les figures 2, 3, 5, 7 et 8, et une deuxième position radiale fléchie , externe, sous contrainte, montrée sur la figure 6, dans laquelle ces pattes 17 sont déformées élastiquement.

Chaque patte 17 présente une tête distale ou zone d'appui 17a, à bords saillants 17b, formant, avec un moyen complémentaire que comprend l'étui 6, à savoir un bossage 38 dans l'exemple représenté, des moyens d'encliquetage permettant d'empêcher la séparation du poussoir 5 et de l'étui 6, après l'engagement du poussoir dans l'étui, comme cela apparaîtra plus loin.

Les pattes 17 comprennent en outre deux paires d'ergots 20, 21 faisant saillie latéralement, situés respectivement sur l'une et l'autre patte à deux hauteurs respectivement différentes. Ces ergots 20, 21 définissent, avec les bossages 38 correspondants aménagés sur l'étui 6, des "points durs" devant être franchis en début de pulvérisation d'une dose, comme cela sera également explicité plus loin.

Chaque patte 17 comprend également une fenêtre ou zone de non appui 22 aménagée au travers d'elle, en retrait d'une zone d'appui 17a ; cette fenêtre s'étend axialement sur une longueur prédéterminée.

Comme le montrent les figures 2 et 4, l'étui 6 comprend deux parois 25, 26 se rejoignant à l'extrémité distale de cet étui, ces parois 25, 26 délimitant entre elles un espace 27 dans lequel le poussoir 5 est destiné à être engagé à coulissement.

La paroi interne 25 délimite un logement 30 de réception et de fixation axiale du corps de seringue 2 et de l'embout de pulvérisation 3, l'étui 6 comprenant des pattes distales 31 qui permettent de centrer l'embout 3 et l'extrémité distale du corps de seringue 2, ainsi que des bossages proximaux 32 d'encliquetage de la collerette 10 du corps de seringue 2.

Cette paroi interne 25 comprend également, dans l'exemple représenté, deux saillies 33 comprenant chacune une rampe oblique, tournée vers l'extrémité distale du dispositif 1, diamétralement opposées, se projetant de sa face extérieure, ces saillies 33 étant destinées à coopérer avec les pattes 17 du poussoir 5, comme cela apparaîtra plus loin. Ces saillies ont par ailleurs une forme adaptée pour pénétrer librement et coulisser à l'intérieur d'une fenêtre 22, comme décrit ci-après.

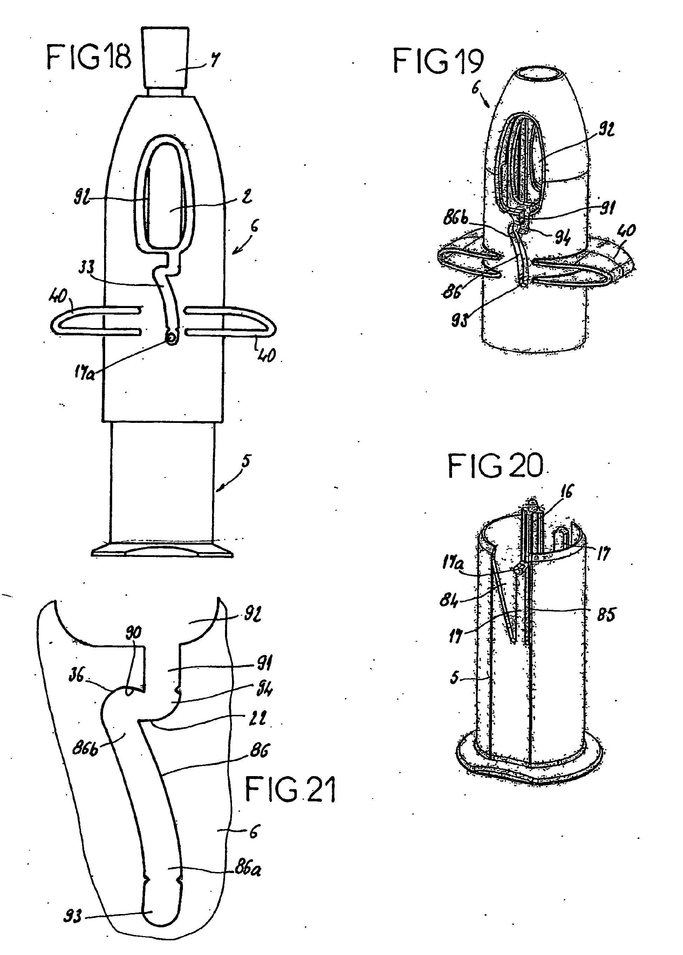

Comme cela apparaît plus distinctement sur la figure 17, les rampes 86 présentent des bords antérieurs 86 c (ceux tournés vers les pattes 17), arrondis, et sont conformées pour n'être rencontrées par les pattes 17 qu'en fin de pulvérisation ou d'injection de la première dose. On évite ainsi la génération de frottement en début de pulvérisation ou d'injection de la première dose, lorsque la vitesse du poussoir est faible

La paroi externe 26 comprend deux retours 35 formant, à l'intérieur dudit espace 27 et à une distance déterminée des saillies 33, des butées internes 36 contre lesquelles les têtes distales 17a des pattes 17 peuvent venir respectivement en appui, comme cela sera également explicité plus loin.

Ces retours 35 comprennent également deux ouvertures 37 qui permettent le passage en translation axiale des pattes 17 à coulissement au travers d'elles.

La paroi externe 26 comprend en outre les deux bossages internes 38, au-delà desquels s'encliquètent les têtes 17a des pattes 17, par leurs bords saillants 17b, pour empêcher la séparation du poussoir 5 et de l'étui 6, et avec lesquels coopèrent les ergots 20, 21 pour former les points durs précités.

La paroi externe 26 comprend également, dans l'exemple représenté, deux pattes externes 40 diamétralement opposées faisant saillie de sa face extérieure, servant à l'appui de deux doigts de l'utilisateur pour l'exercice sur l'étui 6 d'un effort, antagoniste à la poussée exercée sur le fond du poussoir 5 par un autre doigt de l'utilisateur.

Le capuchon 7 présente quant à lui une paroi transversale d'extrémité 45 et une paroi périphérique 46 lui permettant d'envelopper la partie de l'embout 3 dépassant de l'étui 6.

La figure 5 montre le dispositif 1 au début de la phase de pulvérisation d'une première dose de produit. Des efforts antagonistes sont exercés sur le poussoir 5 et sur les pattes externes 40 pour amener l'ergot 20 précité d'une patte 17 à franchir le bossage 38 correspondant, puis pour faire coulisser le poussoir 5 par rapport à l'étui 6, réalisant ainsi la pulvérisation de la première dose.

Au cours de ce déplacement, les têtes ou zones d'appui 17a des pattes 17 viennent rencontrer les rampes 86 des saillies 33, respectivement, ce qui déplace ces pattes 17 vers leurs deuxièmes positions radialement externes précitées, par glissement des têtes 17a, chacune d'une extrémité dite initiale 86a, à une extrémité dite finale 86b d'une rampe 86 ; cf. figure 17.

La poursuite du déplacement du poussoir 5 amène les têtes 17a des pattes 17 à venir en appui contre les butées 36, comme le montre la figure 6, la distance entre chaque butée 36 et chaque saillie 33 correspondante étant telle que cette venue en appui se produit juste avant que la saillie 33 arrive complètement en regard de la fenêtre 22.

La venue en appui des pattes 17 contre les butées 36 maintient les pattes 17 dans ladite deuxième position radiale externe, lorsque les saillies 33 viennent complètement en regard respectivement des fenêtres 22, sous l'effet de la poussée, le coulissement du poussoir 5 demeurant bloqué par rapport à l'étui 6, marquant ainsi la fin de la pulvérisation de la première dose.

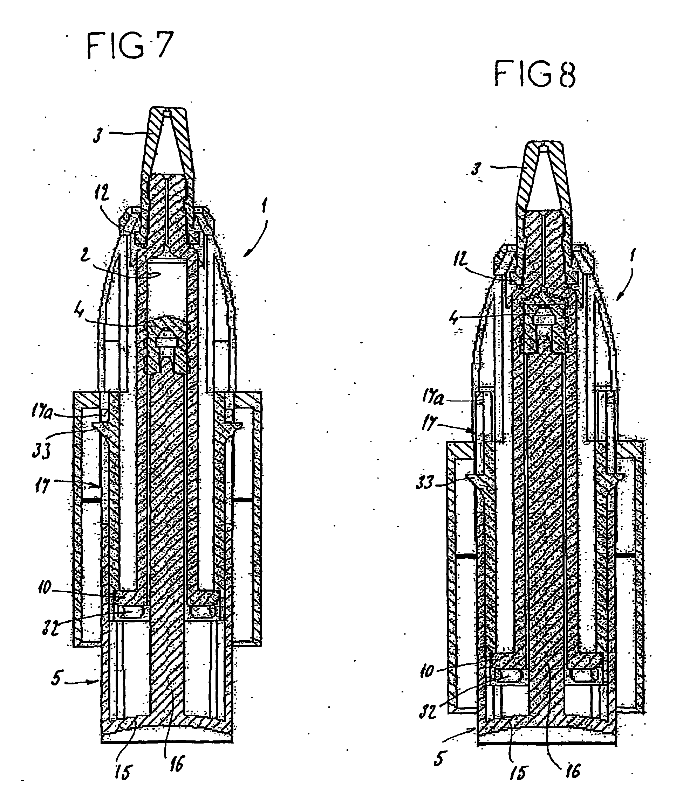

Un relâchement de la poussée exercée sur le poussoir 5, et par conséquent sur les pattes externes 40, provoque élastiquement la libération de cet appui des têtes 17a contre les butées 36, et permet par conséquent le retour des pattes 17 dans leur première position radiale interne, comme montré sur la figure 7.

Une nouvelle poussée peut ensuite être exercée sur le poussoir 5, avec un effort antagoniste sur les pattes externes 40, pour débuter la pulvérisation de la deuxième dose. L'ergot 21 se trouve alors juste en dessous du bossage 38 correspondant, de sorte qu'il existe un deuxième point dur devant être franchi pour débuter cette deuxième pulvérisation. Une fois ce point dur franchi, la deuxième pulvérisation peut se dérouler, les pattes 17 coulissant au travers des ouvertures 37, et les saillies 33 coulissant au travers des fenêtres 22, jusqu'à la position de fin de pulvérisation montrée sur la figure 8.

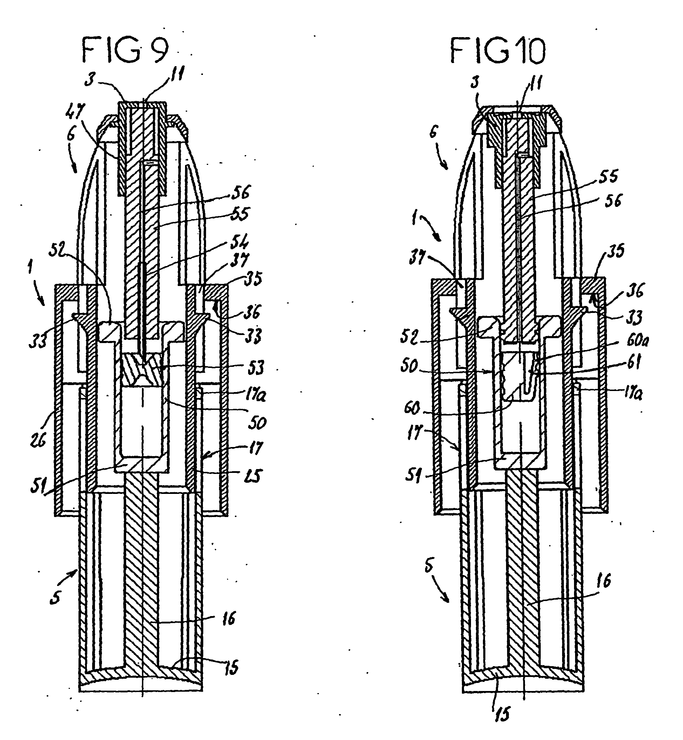

Les figures 9 et 10 montrent un dispositif 1 très similaire à celui qui vient d'être décrit Les éléments déjà décrits en référence aux figures 1 à 8, fonctionnellement identiques ou équivalents, qui se retrouvent dans ce dispositif, sont désignés par les mêmes références numériques et ne seront pas décrits à nouveau.

Dans le cas de la figure 9, le dispositif 1 comprend un récipient 50 du type carpule, à fond proximal 51 et à collerette distale 52, un piston 53 à zone centrale amincie, une aiguille creuse 54 propre à transpercer cette zone centrale amincie, et une tige 55 maintenue par un embout de pulvérisation 3 monté sur l'étui 6, cette tige 55 comportant l'aiguille 54 et formant un conduit 56 d'écoulement du produit.

En pratique, la tige centrale 16 du poussoir 5 appuie contre le fond 51 du récipient 50, ce qui déplace le récipient 50 et le piston 53 jusqu'à ce que l'aiguille 54 transperce la zone centrale amincie du piston 53, permettant ainsi l'écoulement du produit liquide d'intérêt.

Dans le cas de la figure 10, le dispositif 1 comprend un récipient 50 identique à celui du dispositif montré sur la figure 9, et une tige 55 similaire à celle que comprend ce dispositif, mais dépourvue d'aiguille 54. Le piston 60 comprend alors un évidement axial 61, débouchant dans sa face distale et aménagé sur un côté de ce piston 60, cet évidement 61 délimitant ainsi une portion de paroi latérale 60a du piston 60 ayant une souplesse dans le sens radial de ce piston. Cette paroi 60a s'efface sous la pression du produit résultant de l'appui de la tige 55 contre le piston 60, suite au déplacement du récipient 50 par rapport à la tige 55, libérant ainsi l'écoulement du produit.

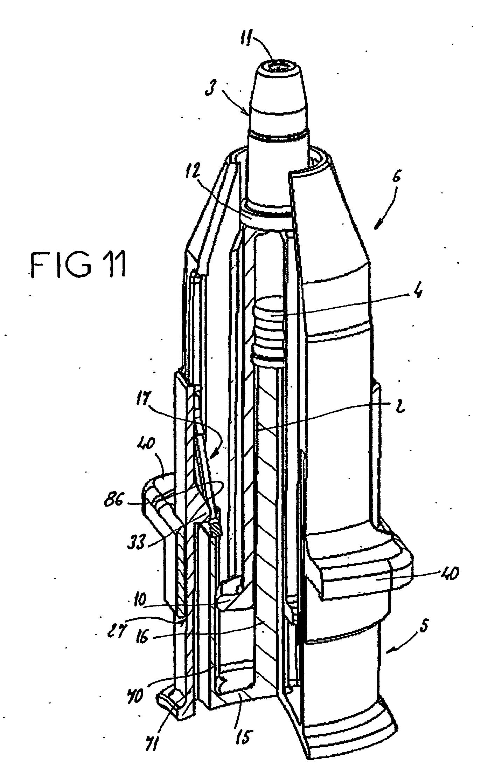

La figure 11 montre un dispositif 1 similaire à celui décrit en référence aux figures 1 à 8, selon une deuxième forme de réalisation de l'invention. Les pattes 17 sont aménagées sur l'étui 6, et les saillies 33 et les butées 36 sont aménagées sur le poussoir 5. Ici également, les éléments structurels, et/ou fonctionnellement identiques ou équivalents, déjà décrits en référence aux figures 1 à 8, qui se retrouvent dans ce dispositif, sont désignés par les mêmes références numériques et ne sont pas à nouveau décrits.

Dans ce cas, le poussoir 5 comprend une paroi interne 70 dans laquelle sont formées les butées 36, et une paroi externe 71, délimitant entre elles les ouvertures 37. La paroi 71 comporte les saillies 33 comportant chacune une rampe 86, et se prolonge au-delà de ces saillies 33, cette paroi 71 étant engagée dans l'espace 27 délimité entre une paroi interne 25 de l'étui 6 et une paroi externe 26 de ce même étui, reliant les pattes 40 entre elles.

Les pattes 17 sont formées par des saignées aménagées de part et d'autre dans ladite paroi interne 25.

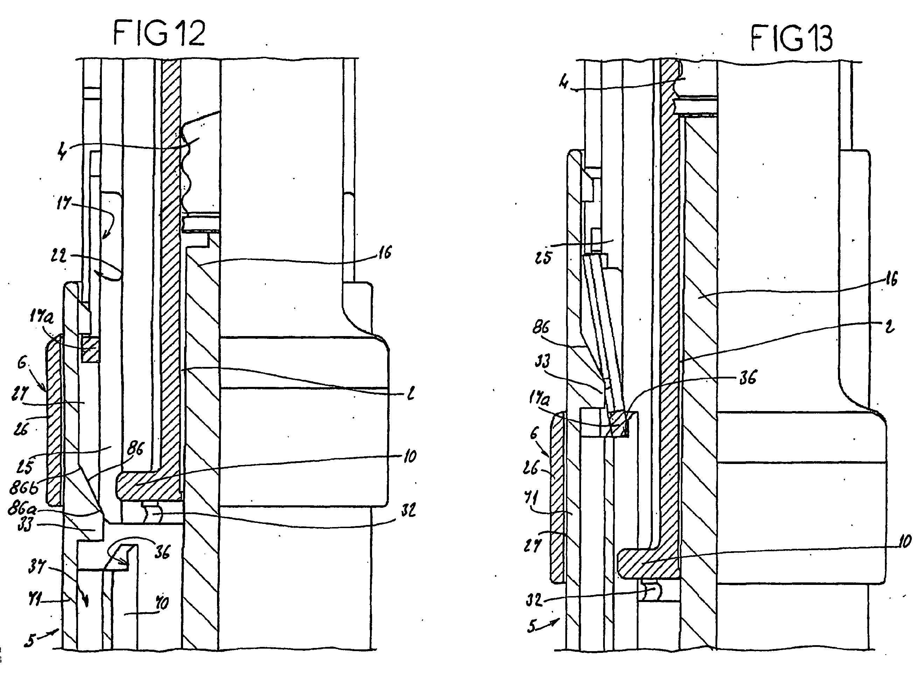

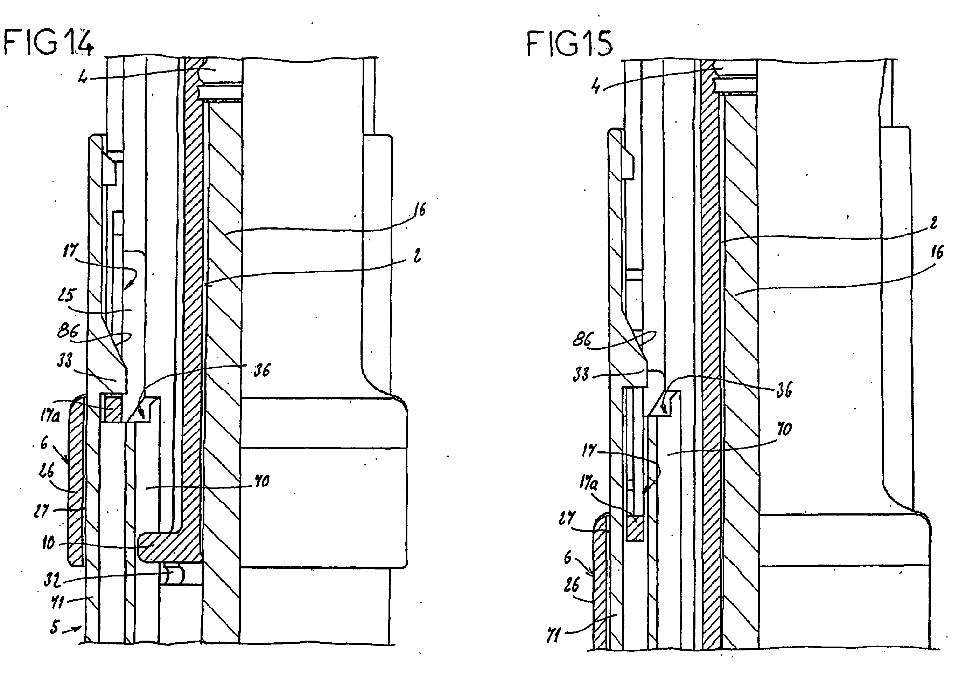

De manière similaire à ce qui a été exposé plus haut, en cours de pulvérisation de la première dose (cf. figure 12), le déplacement du poussoir 5 par rapport à l'étui 6 amène les saillies 33 à déplacer les pattes 17, et plus précisément les têtes 17a par rapport aux rampes 86, vers ladite deuxième position radiale (interne dans ce cas), jusqu'à venue en appui de ces pattes 17 contre les butées 36 (cf. figure 13) ; suite à un relâchement de l'effort exercé sur le poussoir 5, les pattes 17 reviennent dans ladite première position radiale (cf. figure 14), ce qui permet la poursuite la pulvérisation de la deuxième dose, les pattes 17 s'engageant dans les ouvertures 37, et les saillies 33 dans les fenêtres 22, jusqu'à la fin de cette pulvérisation (cf. figure 15).

Les moyens de contrôle de la course de déplacement du poussoir 5 par rapport à l'étui 6, communs aux deux premières formes de réalisation de la présente invention, respectivement selon figures 1 et 11, comprennent donc :
- au moins une patte (17) disposée sur le poussoir (5) ou l'étui (6), mobile entre une première position normale, non contrainte, dans laquelle ladite patte ne fait pas obstacle au déplacement axial du poussoir, et une deuxième position fléchie, sous contrainte, dans laquelle ladite patte arrête le déplacement axial du poussoir ; ladite patte comportant à son extrémité libre une zone ou élément d'appui (17a), agencé pour contribuer à la fois à l'arrêt du déplacement axial du poussoir (5) et au fléchissement de la patte (17) sous l'effet du déplacement axial du poussoir
- au moins une rampe (86) coopérant avec ladite patte d'une extrémité initiale (86a) à une extrémité finale (86b), disposée respectivement sur l'étui (6) ou le poussoir (5), contre laquelle porte la zone ou élément d'appui (17a) de ladite patte (17), dans le sens de la poussée dudit poussoir (5), ladite rampe étant agencée pour amener ladite patte de sa première position normale, à sa deuxième position fléchie,
- au moins une butée (36), coopérant avec la zone ou élément d'appui (17a) de la patte (17), disposée respectivement sur l'étui (6) ou le poussoir (5), respectivement au-delà ou en deçà de l'extrémité finale (86b) de la rampe (86) dans le sens de poussée, contre laquelle la zone ou élément d'appui (17a) de la patte (17) vient finalement en appui, dans sa deuxième position fléchie,
- au moins une zone ou ouverture (22) de non appui, qui est disposée sur la patte (17), en deçà ou au-delà de ladite zone ou élément d'appui (17a), selon que la dite patte est disposée sur le poussoir (5) ou l'étui (6), ladite zone ou ouverture de non appui (22) étant adaptée pour permettre le retour de ladite zone ou élément d'appui (17a), à sa position normale, non contrainte, à partir de la position arrêtée et fléchie de la patte (17), lorsque la poussée sur le poussoir (5) est relâchée,
- la première position de course du poussoir (5) étant déterminée par le déplacement de ce dernier jusqu'à sa positon d'arrêt axial consécutif au contact de la zone ou élément d'appui (17a) avec la butée (36)" et la deuxième portion de course par le déplacement du poussoir, au-delà de la position axiale dans laquelle la zone ou ouverture de non appui (22) a accueilli la zone ou élément d'appui (17a)

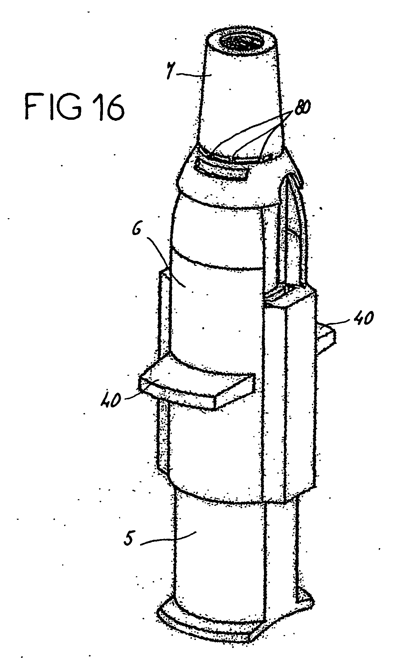

La figure 16 montre un dispositif 1 similaire à la première forme de réalisation de l'invention, sinon qu'il comprend un capuchon 7 relié à l'étui 6 par des ponts ruptibles 80. Ces ponts 80 permettent d'obliger l'utilisateur à avoir une action volontaire d'utilisation et d'éviter que cette utilisation soit trop facile pour des enfants.

Par référence aux figures 18 à 20, on décrit maintenant une troisième forme de réalisation d'un dispositif selon l'invention, se caractérisant en particulier par le fait que la deuxième position fléchie des pattes 17 est obtenue par contrainte tangentielle.

Selon cette troisième forme de réalisation de la présente invention, comme pour la première forme de réalisation précédemment décrite, les pattes 17 sont disposées sur le poussoir 5, tandis que la rampe 86 est disposée sur l'étui 6, sous la forme d'une rainure continue traversant la paroi dudit étui.

L'ouverture 22 de non appui, appartenant à la rainure traversante, est disposée sur l'étui 6, en deçà de la butée 36, appartenant également à ladite rainure traversante, lorsque la patte 17 est disposée sur le poussoir 5 (comme déjà indiqué), et l'élément d'appui 17a précisé ci-après coopère avec la rampe en creux 86, ménagée sur l'étui 6.

Chaque patte 17 disposée sur le poussoir 5 comporte à son extrémité libre un téton 17a, formant élément ou zone d'appui, d'extension transversale vers l'étui 6. Une saignée en forme V, 84, antérieure par rapport à la patte 17 permet le débattement tangentiel de cette dernière, de sa première position normale à sa deuxième position fléchie. La rampe 86 disposée sur l'étui 6 est agencée en creux, ou de manière traversante, pour recevoir le téton 17a, et s'étend sensiblement obliquement (conférer figure 21) dans la paroi de l'étui 6, de l'extrémité initiale 86a à l'extrémité finale 86b. La butée 36, dont il a été question dans les formes d'exécution précédentes de l'invention coopère avec le téton 17a, elle est disposée sur l'étui 6 au-delà de l'extrémité finale 86b de la rampe 86, et elle est déterminée par la jonction en angle, mais continue de la rampe 86 avec un retour de rampe 90, en pente inversée par rapport à la rampe 86 proprement dite. La zone ou ouverture de non appui 22, dont il a été question précédemment, est disposée en creux sur l'étui 6, en deçà de la butée 36 précédemment défini, en continuité avec la rampe 86 et le retour de rampe 90 ; cette zone 22 de non appui est adaptée pour accueillir le téton 17a, lorsque la patte 17 retourne à sa position normale, à partir de sa position arrêtée et fléchie. Et une fente axiale 91 s'étend en continuité à partir de la zone de non appui 22 précédemment décrite, mais au-delà de cette dernière. La fente axiale 91 dans l'étui 6 débouche librement dans une ouverture traversante 92 de l'étui 6, formant fenêtre au travers de la paroi extérieure dudit étui ; étant entendu que, selon cette troisième forme d'exécution de l'invention, la deuxième portion de course du poussoir 5 est déterminée par la butée du piston contre la paroi du récipient 2.

En deçà de l'extrémité initiale 86a de la rampe 86, et en continuité avec cette dernière, un logement de stationnement 93 du téton 17 est ménagé. L'encliquetage du téton 17a dans le logement 93 permet l'assemblage définitif du poussoir 5 et de l'étui 6.

Au-delà de la zone ou ouverture de non appui 22, et en continuité avec cette dernière, un logement de stationnement 94 du téton 17a est ménagé, comme pour le logement 93, dans la paroi extérieure de l'étui 6.

La troisième forme de réalisation de l'invention, précédemment décrite, apporte une plus grande robustesse ainsi qu'une dimension axiale moindre. Cette forme de réalisation garantit également un arrêt effectif du poussoir par rapport à l'étui entre les deux portions de la course dudit poussoir, par conséquent avant que la deuxième dose du produit d'intérêt soit délivré.

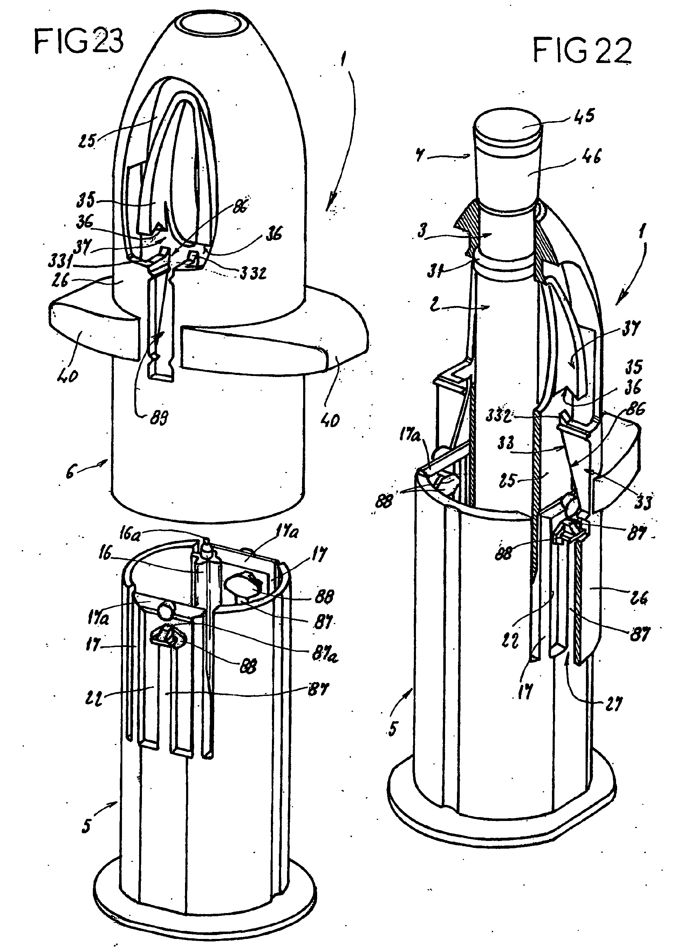

Par référence aux figures 22 à 30, on décrit maintenant une quatrième et dernière forme de réalisation de la présente invention, qui s'apparente à la première ou à la deuxième forme de réalisation précédemment décrite.

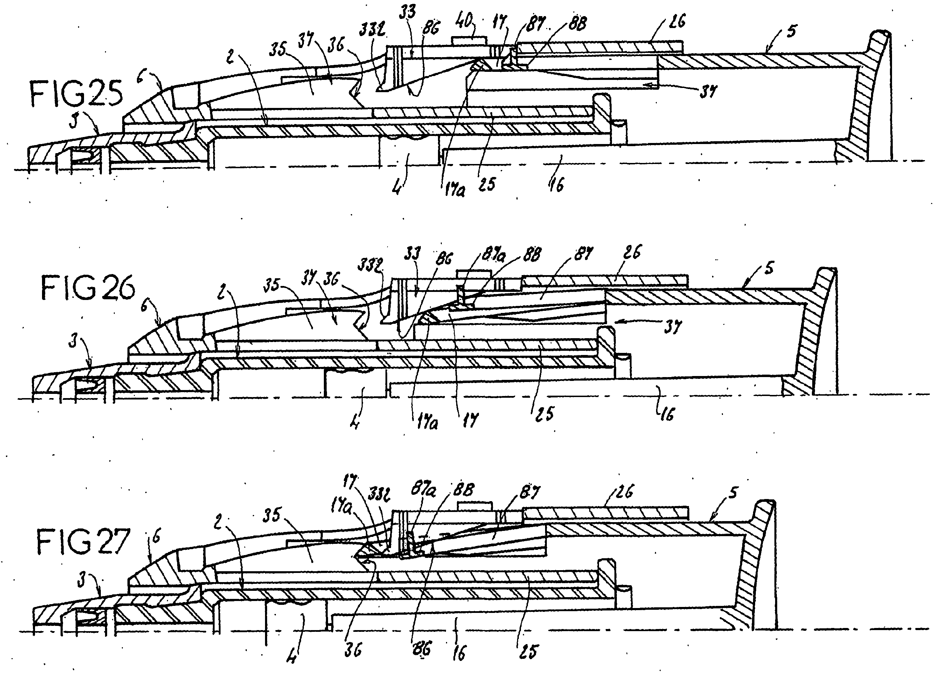

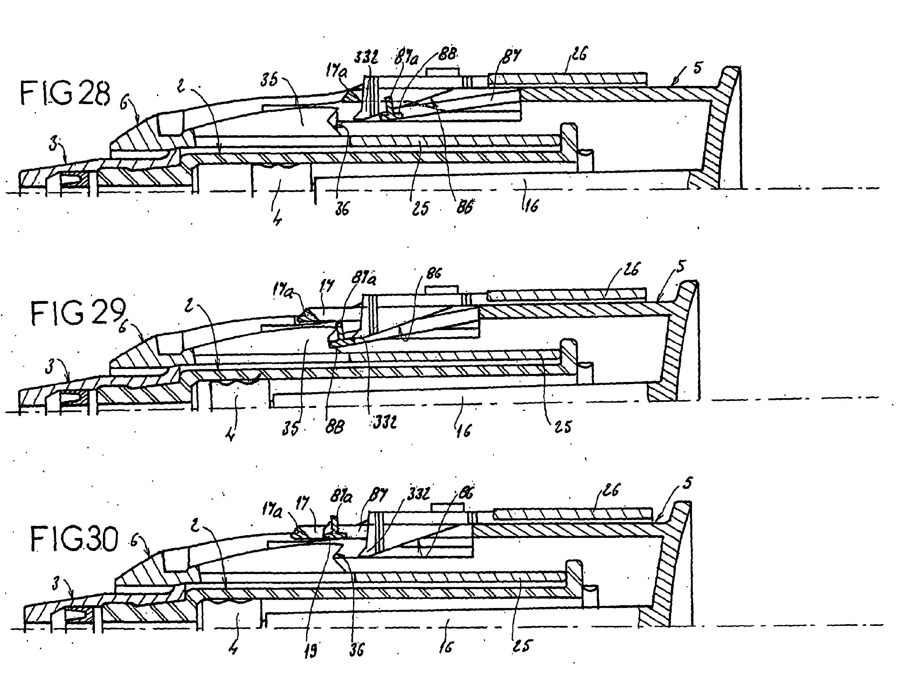

Au moins une languette 87, distincte ou indépendante de la patte 17, est disposée selon le cas sur le poussoir 5 ou l'étui 6, selon que la patte 17 est elle-même disposée sur le poussoir 5 ou l'étui 6, respectivement. Cette languette 87 est mobile entre, une première position normale, non contrainte, dans laquelle ladite languette ne fait pas obstacle, de manière générale, au déplacement axial du poussoir pendant les deux portions de course successives de ce dernier, et une deuxième position fléchie, sous contrainte, dans laquelle ladite languette 87 contribue au rappel du poussoir 5, en sens inverse de la poussée, et ce pour permettre le retour de la patte ou des pattes 17 à leur position normale, à partir de leur position arrêtée et fléchie. Comme montré en perspective par la figure 23, mais plus explicitement par les figures 25 à 30, la rampe 86 et l'extrémité libre 87a de la languette 87 sont adaptées pour coopérer l'une avec l'autre, en sorte que pendant la première portion de course du poussoir, par exemple à la fin de cette dernière, la languette 87 se trouve fléchie (conférer figure 27), et pendant la deuxième portion de course du poussoir, la languette 87 échappe à la rampe 86 et retourne à sa position normale (conférer figures 29 et 30). La première position normale, non contrainte, de la languette 87 est représentée figure, 25, tandis que une deuxième position fléchie, sous contrainte, est représentée aux figures 26 à 28.

Selon les figures 22 à 30, la deuxième position fléchie de la languette 87 est obtenue par contrainte radiale, lorsque la deuxième position fléchie des pattes 17 est elle-même obtenue par contrainte radiale également. Il doit être entendu que, de manière non représentée, cette deuxième position fléchie de la languette 87 pourrait être obtenue par contrainte tangentielle, alors que la deuxième position fléchie des pattes 17 serait elle-même obtenue par contrainte tangentielle.

Chaque patte 17, disposée selon le cas sur le poussoir 5 ou l'étui 6, comprend une fenêtre 22 de forme générale de forme rectangulaire, de non appui, au centre de laquelle s'étend la languette 87 parallèlement à l'axe du dispositif. Comme pour les autre formes de réalisation de l'invention, la rampe 86 appartient à une saillie 33 disposée selon le cas sur l'étui 6 ou le poussoir 5, laquelle est adaptée pour traverser librement la fenêtre 22. La butée 36 est agencée, sur l'étui 6 ou le poussoir 5, selon le cas, en sorte que dans la deuxième position fléchie de chaque patte 17, la saillie 33 vient en regard de la fenêtre 22, puis pénètre au travers de cette dernière, lorsque la poussée sur le poussoir 5 est relâchée, ce qui provoque le retour de chaque patte 17 dans sa première position.

Plus précisément, chaque languette 87 est disposée au centre de la fenêtre 22, avec son extrémité libre 87a demeurant en deçà de la tête 17a ou zone d'appui de la patte correspondante. L'extrémité libre 87a comprend deux oreilles 88 d'appui contre la rampe 86. Cette dernière comprend deux ailes parallèles 331 et 332, disposées de part et d'autre d'une fente 89, laquelle permet le libre passage en translation de la languette 87. Chaque oreille 88 est agencée pour venir en contact d'une portion de rampe 86 définie par une aile 331 ou 332, et ce dans le sens de la poussée du poussoir 5 ; au-delà de la première portion de course du poussoir 5, chaque oreille 88 échappe à la portion de rampe ou ailes 331 ou 332, correspondante, en sorte que la languette 87 revient en position normale.

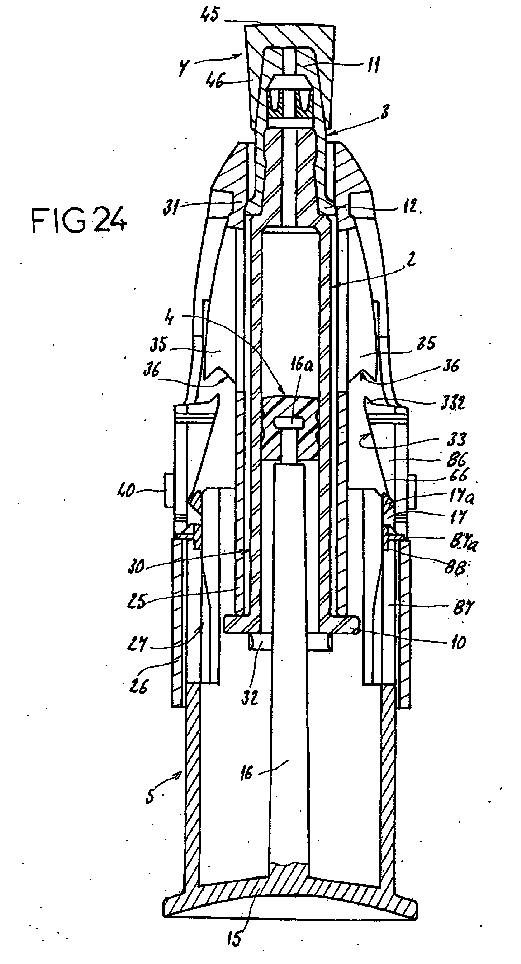

Dans la position initiale du poussoir 5 par rapport à l'étui 6, représentée à la figure 24, la patte 17 et la languette 87 sont dans leur position normale, radiale, et non contrainte.

Conformément à la figure 26, par poussée du poussoir 5, le déplacement de la tête 17a de la patte 17 provoque le fléchissement de cette dernière, sans provoquer pour autant, au début, le fléchissement de la languette 87 correspondante, ce fléchissement intervient en fin de course de la tête 17a.

Conformément à la figure 27, en poursuivant le déplacement du poussoir 5, la patte 17 est fléchie complètement alors que les oreilles 88 au contact de la rampe 33, et plus précisément respectivement des ailes 331 et 332, fléchissant la languette 87 correspondante, grâce à l'ouverture ou zone de non appui 22, en poursuivant la poussée du poussoir 5, la patte 17 échappe à la rampe 86, ou saillie 33, la languette 87 correspondante demeurant fléchie, la languette 87 fléchie exerce contre la rampe 86 une poussée antagoniste, contribuant au rappel du poussoir 5, en sens inverse de la poussée, lorsque cette dernière est relâchée. Dès lors la languette 87 reprend une position intermédiaire, fléchie ; cf. figure 28

A partir de la position relative de l'étui et du poussoir représentés, à la figure 28, en reprenant la poussée du poussoir 5, l'extrémité libre 87a de la languette 87 au contact de la rampe 86, échappe immédiatement à cette dernière (cf. figure 29), en sorte que le poussoir 5 peut être poussé au-delà de la position relative montrée par la figure 28, pour délivrer la deuxième dose du produit d'intérêt ; cf. figure 30.

Ainsi qu'il apparaît de ce qui précède, l'invention apporte une amélioration déterminante à la technique antérieure, en fournissant un dispositif à la fois aisé à manipuler, réduisant au minimum les erreurs d'utilisation et restant d'une structure relativement simple et peu onéreuse à fabriquer.

Il va de soi que l'invention n'est pas limitée à la forme de réalisation décrite ci-dessus à titre d'exemple mais qu'elle en embrasse au contraire toutes les variantes de réalisation entrant dans le champ de protection défini par les revendications ci-annexées. Le corps de seringue 2 et l'embout de pulvérisation 3 peuvent notamment être fabriqués en une seule pièce.

## Revendications

1. Dispositif (1) de pulvérisation ou d'injection permettant de délivrer au moins deux doses déterminées de produit, comprenant :
- un récipient (2, 50) contenant le produit à pulvériser ou à injecter,
- un piston (4, 53, 60) placé dans ce récipient (2, 50) et déplaçable dans ce récipient (2, 50) pour extraire le produit hors du récipient (2, 50),
- un étui (6) dans lequel est placé le récipient (2, 50),
- un poussoir (5) déplaçable par rapport à l'étui (6) pour permettre le déplacement relatif du récipient (2, 50) et du piston (4, 53, 60), et
- des moyens (17, 33, 36, 37) permettant de subdiviser la course de déplacement du poussoir (5) par rapport à l'étui (6) en au moins deux portions de course, correspondant chacune à la délivrance d'une dose de produit ;
dispositif (1) **caractérisé en ce que** :
- l'étui (6) ou le poussoir (5) comprend au moins une patte (17) mobile radialement entre une première position radiale normale, dans laquelle la patte (17) ne fait pas obstacle au déplacement du poussoir (5) par rapport à l'étui (6), et une deuxième position radiale, dans laquelle la patte (17) fait obstacle à ce déplacement, cette patte (17) comprenant une zone d'appui (17a) et une zone de non appui (22) ;
- le poussoir (5) ou l'étui (6), respectivement, comprend au moins une saillie (33) en forme de rampe, contre laquelle vient porter ladite zone d'appui (17a) de la patte (17) au cours du déplacement du poussoir (5) par rapport à l'étui (6) dans le sens permettant la pulvérisation ou l'injection du produit, ce qui amène ladite patte (17) dans ladite deuxième position radiale, puis en regard de laquelle vient ladite zone de non appui (22) de la patte (17), ce qui permet le retour de ladite patte (17) dans ladite première position radiale ; le poussoir (5) ou l'étui (6), respectivement, comprend en outre au moins une zone de butée (36) contre laquelle la patte (17) vient en appui lorsqu'elle est amenée dans ladite deuxième position radiale par ladite saillie (33), cet appui se produisant juste avant que ladite saillie (33) vienne en regard de ladite zone de non appui (22),
le dispositif (1) étant conçu de telle sorte que le relâchement de l'effort exercé sur le poussoir (5), qui permet de déplacer ce poussoir (5) par rapport à l'étui (6), permette de libérer l'appui de la patte (17) contre ladite zone de butée (36) et donc permette le retour de ladite patte (17) dans ladite première position radiale, ladite saillie (33) venant alors en regard de ladite zone de non appui (22), ce retour de ladite patte (17) dans ladite première position radiale permettant de libérer le déplacement du poussoir (5) pour une portion de course de déplacement suivante, en vue de la pulvérisation ou de l'injection d'une dose suivante du produit.

2. Dispositif (1) selon la revendication 1, **caractérisé en ce que** ladite zone de non appui de la patte (17) est formée par une fenêtre (22) aménagée dans la patte (17) en retrait de l'extrémité de cette patte, ladite zone d'appui étant formée par la zone (17a) de cette patte reliant cette extrémité de la patte et cette fenêtre (22).

3. Dispositif (1) selon la revendication 1 ou la revendication 2, **caractérisé en ce qu'**il comprend au moins deux pattes (17), deux saillies (33) et deux zones de butée (36) diamétralement opposées.

4. Dispositif (1) selon l'une des revendications 1 à 3, **caractérisé en ce que** l'étui (6) et le poussoir (5) comprennent des moyens (20, 21, 38) permettant de former au moins un "point dur" devant être franchi en début de délivrance d'une dose.

5. Dispositif (1) selon la revendication 4, **caractérisé en ce que** lesdits moyens permettant de former au moins un "point dur" sont constitués par au moins un ergot (20, 21) faisant saillie latéralement d'une patte (17) telle que précitée et par au moins un bossage (38) aménagé à un endroit adéquat correspondant de l'étui (6).

6. Dispositif (1) selon l'une des revendications 1 à 5, **caractérisé en ce que** l'ensemble du poussoir (5) est réalisé en une matière plastique courante, légèrement flexible, la mobilité de chaque patte 17 résultant de la flexibilité de ce matériau, chaque patte (17) n'étant pas déformée dans ladite première position radiale et étant déformée élastiquement dans ladite deuxième position radiale.

7. Dispositif (1) selon l'une des revendications 1 à 6, **caractérisé en ce que** chaque patte (17) présente une tête distale (17a) à bords saillants, formant, avec un moyen complémentaire que comprend l'étui (6), des moyens d'encliquetage permettant d'empêcher la séparation du poussoir (5) et de l'étui (6) après l'engagement du poussoir dans l'étui.

8. Dispositif (1) selon l'une des revendications 1 à 7, **caractérisé en ce que** l'étui (6) comprend deux parois (25, 26) délimitant entre elles un espace (27) dans lequel le poussoir (5) est destiné à être engagé à coulissement, chaque saillie (33) se projetant de la face extérieure de la paroi interne (25) et la paroi externe (26) comprenant au moins un retour (35) formant, à l'intérieur dudit espace (27) et à une distance déterminée de la saillie (33), ladite zone de butée (36), chaque retour (35) comprenant en outre une ouverture (37) qui permet le passage de la patte (17) à coulissement au travers d'elle.

9. Dispositif (1) selon l'une des revendications 1 à 7, **caractérisé en ce que** l'étui (6) comprend deux parois (25, 26) délimitant entre elles un espace (27) dans lequel le poussoir (5) est destiné à être engagé à coulissement, le poussoir (5) comprenant une paroi interne (70), dans laquelle est aménagée chaque zone de butée (36), et une paroi externe (71) comportant chaque saillie (33), cette paroi externe (71) se prolongeant au-delà de chaque saillie (33) et étant engagée dans ledit espace (27), lesdites parois interne (70) et externe (71) du poussoir (5) délimitant entre elles chaque ouverture (37) ; chaque patte (17) est aménagée dans ladite paroi interne (25) de l'étui (6).

10. Dispositif (1) selon l'une des revendications 1 à 9, **caractérisé en ce que** le récipient est formé par un corps de seringue (2) comprenant une collerette proximale (10) et un conduit distal d'écoulement, ce corps de seringue (2) comportant un embout de pulvérisation (3) emmanché sur son extrémité distale, qui forme une tête de pulvérisation (11) permettant de pulvériser le produit contenu dans le corps de seringue (2).

11. Dispositif (1) selon l'une des revendications 1 à 9, **caractérisé en ce qu'**il comprend un récipient (50) à fond proximal (51), un piston (53) à zone centrale amincie, une aiguille creuse (54) propre à transpercer cette zone centrale amincie, et une tige (55) maintenue sur l'étui (6), cette tige (55) comportant l'aiguille (54) et formant un conduit (56) d'écoulement du produit.

12. Dispositif (1) selon l'une des revendications 1 à 9, **caractérisé en ce qu'**il comprend un récipient (50) à fond proximal (51), un piston (60) comprenant un évidement axial (61) qui débouche dans la face distale du piston (60) et qui est aménagé sur un côté de ce piston (60), cet évidement (61) délimitant ainsi une portion de paroi latérale (60a) de ce piston (60) ayant une souplesse dans le sens radial du piston, et une tige (55) maintenue sur l'étui (6), formant un conduit (56) d'écoulement du produit, cette tige (55) étant propre à appuyer contre le piston (60) au cours du déplacement du poussoir (5) par rapport à l'étui (6).

13. Dispositif (1) de pulvérisation ou d'injection d'un produit d'intérêt sous forme liquide, permettant de délivrer successivement au moins une première dose et une deuxième dose prédéterminées, dudit produit, ledit dispositif s'étendant selon un axe de référence d'une extrémité distale à une extrémité proximale, comprenant :
- un récipient allongé axialement (2, 50) contenant ledit produit,
- un piston (4, 53, 60) placé dans le récipient, et obturant ce dernier, ledit piston étant déplaçable axialement par rapport au récipient, dans un sens de référence, permettant la propulsion distale dudit produit hors du récipient,
- un étui (6) agencé pour recevoir et y fixer axialement ledit récipient,
- un poussoir (5) assemblé avec l'étui (6), déplaçable par rapport à ce dernier, agencé pour venir en butée axiale et proximale contre le récipient ou le piston, et déplacer ledit piston dans le sens de référence,
- des moyens de contrôle (17, 33, 36, 37) de la course de déplacement du poussoir par rapport à l'étui, agencés pour diviser cette dernière en une première portion de course et une deuxième portion de course, déterminant la délivrance respectivement des première et deuxième doses, ledit dispositif étant **caractérisé en ce que** l'étui (6) et le poussoir (5) sont agencés pour être déplaçables axialement l'un par rapport à l'autre dans un sens de poussée, générant le déplacement du piston dans le sens de référence, et
**en ce que** les moyens de contrôle comprennent :
- au moins une patte (17) disposée sur le poussoir (5) ou l'étui (6) mobile entre une première position normale, non contrainte, dans laquelle ladite patte ne fait pas obstacle au déplacement axial du poussoir, et une deuxième position fléchie, sous contrainte, dans laquelle ladite patte arrête le déplacement axial du poussoir, ladite patte comportant à son extrémité libre un zone ou élément d'appui (17a) agencé pour contribuer à la fois à l'arrêt du déplacement axial du poussoir (5) et au fléchissement de la patte (17) sous l'effet du déplacement axial du poussoir,
- au moins une rampe (86) coopérant avec ladite patte d'une extrémité dite initiale (86a) à une extrémité dite finale (86b), disposée respectivement sur l'étui (6) ou le poussoir (5), contre laquelle porte la zone ou élément d'appui (17a) de ladite patte (17), dans le sens de la poussée dudit poussoir (5), ladite rampe étant agencée pour amener ladite patte de sa première position normale, à sa deuxième position fléchie,
- au moins une butée (36), coopérant avec la zone ou élément d'appui (17a) de la patte (17), disposée respectivement sur l'étui (6) ou le poussoir (5), respectivement au-delà ou en deçà de l'extrémité finale (86b) de la rampe (86) dans le sens de la poussée, contre laquelle la zone ou élément d'appui (17a) de la patte (17) vient finalement en contact dans sa deuxième position fléchie,
- au moins une zone ou ouverture (22) de non appui, qui est, soit disposée sur la patte (17), en deçà ou au-delà de ladite zone ou élément d'appui (17a), selon que ladite patte est disposée sur le poussoir (5) ou l'étui (6), soit disposée sur l'étui (6), en deçà de la butée (36), lorsque la patte (17) est disposée sur le poussoir (5) et l'élément d'appui (17a) coopère avec une rampe en creux (86) ménagée sur l'étui, ladite zone ou ouverture de non appui (22) étant adaptée pour permettre le retour de ladite zone ou élément d'appui (17a), à sa position normale, non contrainte, à partir de la position arrêtée et fléchie de la patte 17, lorsque la poussée sur le poussoir (5) est relâchée,
- la première portion de course du poussoir (5) étant déterminée par le déplacement de ce dernier jusqu'à sa position d'arrêt axial, consécutif au contact de la zone ou élément d'appui (17a) avec la butée (36), et la deuxième portion de course par le déplacement du poussoir, au-delà de la position axiale dans laquelle la zone ou ouverture de non appui (22) a accueilli la zone ou élément d'appui (17a).

14. Dispositif selon la revendication 13, **caractérisé en ce que** la deuxième position fléchie de la patte est obtenue par contrainte radiale.

15. Dispositif selon la revendication 14, **caractérisé en ce que** la patte (17), disposée sur le poussoir (5) ou l'étui (6), comprend une fenêtre (22) de non appui, en retrait par rapport à ladite zone ou élément d'appui (17a) ; la rampe (86) appartient à une saillie (33), disposée respectivement sur l'étui (6) ou le poussoir (5), et adaptée pour traverser librement ladite fenêtre (22) ; la butée (36) est agencée en sorte que, dans la deuxième position fléchie de ladite patte, la saillie (33) vient en regard de la fenêtre (22)-de la patte, puis pénètre au travers de ladite fenêtre, lorsque la poussée sur le poussoir (5) est relâchée, ce qui provoque le retour de ladite patte dans sa première position.

16. Dispositif selon la revendication 13, **caractérisé en ce que** la deuxième position fléchie de la patte est obtenue par contrainte tangentielle.

17. Dispositif selon la revendication 16, **caractérisé en ce que** la patte (17) est disposée sur le poussoir (5), comporte à son extrémité libre un téton (17a) d'extension transversale vers l'étui (6), une saignée antérieure (84, 85) à ladite patte permettant son débattement tangentiel de sa première position normale à sa deuxième position fléchie, la rampe (86) est disposée sur l'étui (6), agencée en creux pour recevoir le téton (17a), et s'étend sensiblement obliquement dans la paroi de l'étui, de l'extrémité initiale (86a) à l'extrémité finale (86b) ; la butée (36), coopérant avec le téton (17a), disposée sur l'étui (6) au-delà de l'extrémité finale (86b) de la rampe (86) est déterminée par la jonction en angle, mais continue, de ladite rampe (86) avec un retour de rampe (90) ; la zone ou ouverture de non appui (22), disposée en creux sur l'étui (6) en-deçà de la butée (36), est adaptée pour accueillir le téton (17a), lorsque la patte (17) retourne à sa position normale ; et une fente axiale (91) s'étend, en continuité de la zone de non appui (22), au-delà de cette dernière.

18. Dispositif selon la revendication 17, **caractérisé en ce que** la fente axiale (91) débouche librement dans une ouverture traversante (92) de l'étui (6), la deuxième portion de course du poussoir (5) étant déterminée par la butée du piston (4) contre la paroi du récipient (2).

19. Dispositif selon la revendication 17, **caractérisé en ce que** en-deçà de l'extrémité initiale (86a) de la rampe (86), et en continuité avec cette dernière, un logement de stationnement (93) du téton (17a) est ménagé, notamment pour permettre l'assemblage définitif du poussoir (5) et de l'étui (6).

20. Dispositif selon la revendication 17, **caractérisé en ce que**, au-delà de la zone ou ouverture de non appui (22), et en continuité avec cette dernière, un logement de stationnement (94) du téton (17a) est ménagé.

21. Dispositif selon la revendication 13, **caractérisé en ce que**, d'une part il comprend au moins une languette (87), distincte ou indépendante de la patte (17), disposée sur le poussoir (5) ou l'étui (6), selon que la patte (17) est disposée sur le poussoir (5) ou l'étui (6), mobile entre une première position normale, non contrainte, dans laquelle ladite languette ne fait pas obstacle au déplacement axial du poussoir (5), et une- deuxième position fléchie, sous contrainte, dans laquelle ladite languette (87) contribue au rappel du poussoir (5), pour permettre le retour de ladite patte (17) à sa position normale, à partir de sa position arrêtée et fléchie, et d'autre part, la rampe (86) et l'extrémité libre (87a) de la languette (87) sont adaptées pour coopérer l'une avec l'autre, en sorte que, pendant la première portion de course du poussoir, la languette se trouve fléchie, et pendant la deuxième portion de course du poussoir, la languette (87) échappe à la rampe et retourne à sa position normale.

22. Dispositif selon la revendication 21, **caractérisé en ce que** la deuxième position fléchie de languette (87) est obtenue par contrainte radiale, lorsque la deuxième position fléchie de la patte (17) est obtenue par contrainte radiale.

23. Dispositif selon la revendication 21, **caractérisé en ce que** la deuxième position fléchie de languette (87) est obtenue par contrainte tangentielle, lorsque la deuxième position fléchie de la patte (17) est obtenue par contrainte tangentielle.

24. Dispositif selon la revendication 22, **caractérisé en ce que** la patte (17), disposée sur le poussoir (5) ou l'étui (6), comprend une fenêtre (22) de non appui, la rampe (86) appartient à une saillie (33), disposée respectivement sur l'étui (6) ou le poussoir (5), et adaptée pour traverser librement ladite fenêtre (22) ; la butée (36) est agencée en sorte que, dans la deuxième position fléchie de ladite patte, la saillie (33) vient en regard de la fenêtre (22) de la patte, puis pénètre au travers de ladite fenêtre, lorsque la poussée sur le poussoir (5) est relâchée, ce qui provoque le retour de ladite patte dans sa première position.

25. Dispositif selon la revendication 24, **caractérisé en ce que**, d'une part la languette (87) est disposée au centre de la fenêtre (22), l'extrémité libre (87a) de la languette (87) demeurant en-deçà de la tête (17a), et comprenant deux oreilles (88) d'appui contre la rampe (86), et d'autre part la rampe (90) comprend deux ailes (331) et (332) disposées de part et d'autre d'une fente (89) permettant le libre passage en translation de la languette, chaque oreille (88) venant au contact d'une portion de rampe (86) définie par une aile (331, 332), dans le sens de la poussée du poussoir (5), pour échapper à ladite portion de rampe, au-delà de la première portion de course du poussoir (5).

## Patentansprüche

1. Sprüh- oder Injektionsvorrichtung (1) zur Abgabe mindestens zweier vorbestimmter Dosen eines Produkts, welche Folgendes aufweist:
- ein Behältnis (2, 50), welches das zu versprühende oder zu injizierende Produkt enthält,
- einen Kolben (4, 53, 60), welcher in dem Behältnis (2, 50) angeordnet und in dem Behältnis (2, 50) verschiebbar ist, um das Produkt aus dem Behältnis (2, 50) herauszudrücken,
- eine Hülle (6), in welcher das Behältnis (2, 50) angeordnet ist,
- einen Stößel (5), welcher im Verhältnis zur Hülle (6) verschiebbar ist, um eine Verschiebung relativ zum Behältnis (2, 50) und zum Kolben (4, 53, 60) zu ermöglichen, sowie
- Mittel (17, 33, 36, 37), durch welche der Verlauf der Verschiebung des Stößels (5) im Verhältnis zur Hülle (6) in zumindest zwei Teilverlaufe unterteilt werden kann, welche jeweils der Abgabe einer Dosis des Produkts entsprechen;
wobei die Vorrichtung (1) **dadurch gekennzeichnet ist, dass**
- die Hülle (6) oder der Stößel (5) zumindest eine Lasche (17) aufweisen, welche zwischen einer ersten radialen normalen Position, in welcher die Lasche (17) die Verschiebung des Stößels (5) im Verhältnis zur Hülle (6) nicht blockieren kann, und einer zweiten radialen Position radial beweglich ist, in welcher die Lasche (17) diese Verschiebung blockieren kann, wobei die Lasche (17) einen aufliegenden Bereich (17a) und einen nicht-aufliegenden Bereich (22) aufweist;
- der Stößel (5) oder die Hülle (6) jeweils zumindest einen Vorsprung (33) in Form einer Schräge umfassen, gegen welche der aufliegende Bereich (17a) der Lasche (17) stößt, und zwar im Verlauf der Verschiebung des Stößels (5) im Verhältnis zur Hülle (6) in die Richtung, welche das Versprühen oder Injizieren des Produktes ermöglicht, was die Lasche (17) in eine zweite radiale Position führt, gegenüber welcher dann der aufliegende Bereich (22) der Lasche (17) zu liegen kommt, was das Zurückführen der Lasche (17) in die erste radiale Position ermöglicht; der Stößel (5) oder die Hülle (6) umfassen jeweils zumindest einen Anschlagsbereich (36), gegen welchen die Lasche (17) in Auflage kommt, wenn sie über den Vorsprung (33) in die zweite radiale Position geführt wird, wobei die Auflage, die genau vor dem Vorsprung (33) erscheint, gegenüber der nicht-aufliegenden Zone (22) zu liegen kommt,
- die Vorrichtung (1) derart ausgebildet ist, dass das Nachlassen der auf den Stößel (5) ausgeübten Kraft, welche die Verschiebung des Stößels (5) im Verhältnis zur Hülle (6) ermöglicht, die Freisetzung der Auflage der Lasche (17) gegen den Anschlagsbereich (36) ermöglicht, und daher das Rückführen der Lasche (17) in die erste radiale Position ermöglicht, wobei der Vorsprung (33) dann gegenüber dem Bereich, der nicht aufliegt (22), zu liegen kommt, und wobei diese Rückführung der Lasche (17) in die erste radiale Position es ermöglicht, die Verschiebung des Stößels (5) für einen Teilverlauf der folgenden Verschiebung freizugeben, im Hinblick auf das Versprühen oder die Injizierung einer folgenden Dosis des Produkts.

2. Vorrichtung (1) nach Anspruch 1, **dadurch gekennzeichnet, dass** der nicht aufliegende Bereich der Lasche (17) durch ein Fenster (22) gebildet ist, welches in der Lasche (17) ausgebildet ist, und vom Kopfende dieser Lasche zurückgesetzt ist, und wobei der aufliegende Bereich durch einen Bereich (17a) dieser Lasche gebildet wird, welcher das Kopfende der Lasche und das Fenster (22) miteinander verbindet.

3. Vorrichtung (1) nach Anspruch 1 oder Anspruch 2, **dadurch gekennzeichnet, dass** sie zumindest zwei Laschen (17), zwei Vorsprünge (33) und zwei Anschlagbereiche (36) umfasst, welche einander diametral gegenüberliegen.

4. Vorrichtung (1) nach einem der Ansprüche 1 bis 3, **dadurch gekennzeichnet, dass** die Hülle (6) und der Stößel (5) Mittel (20, 21, 38) umfassen, welche die Bildung von zumindest einem "harten Punkt" ermöglichen, der überwunden werden muss, um mit der Abgabe einer Dosis beginnen zu können.

5. Vorrichtung (1) nach Anspruch 4, **dadurch gekennzeichnet, dass** die Mittel, welche die Bildung von zumindest einem "harten Punkt" ermöglichen, aus zumindest einer Noppe (20, 21), welche den seitlichen Vorsprung der oben genannten Lasche (17) ausmacht, und aus zumindest einer Erhebung (38) besteht welche einer passenden, der Hülle (6) entsprechenden Stelle zugeführt wird.

6. Vorrichtung (1) nach einem der Ansprüche 1 bis 5, **dadurch gekennzeichnet, dass** der Stößel (5) in seiner Gesamtheit aus einem harten, leicht flexiblen Plastikmaterial hergestellt ist, und dass die Beweglichkeit jeder Lasche (17) von der Flexibilität des Materials herrührt, wobei jede Lasche (17) in der ersten radialen Position nicht verformt ist und in der zweiten radialen Position elastisch eingebogen ist.

7. Vorrichtung (1) nach einem der Ansprüche 1 bis 6, **dadurch gekennzeichnet, dass** jede Lasche (17) einen distalen Kopf (17a) mit vorspringenden Rändern aufweist, welche mit einem von der Hülle (6) umfassten komplementären Mittel Einrastmittel bilden, die die Trennung des Stößels (5) und der Hülle (6) nach dem Eingriff des Stößels in die Hülle verhindern können.

8. Vorrichtung (1) nach einem der Ansprüche 1 bis 7, **dadurch gekennzeichnet, dass** die Hülle (6) zwei Wände (25, 26) aufweist, welche zwischen einander einen Raum (27) begrenzen, in welchen der Stößel (5) verschiebbar eingreifen soll, wobei jeweils der Vorsprung (33) von der äußeren Fläche der Innenwand (25) vorsteht, und die Außenwand (26) zumindest einen Rückhalt (35) aufweist, welcher - im Inneren des Raumes (27) und in einem bestimmten Abstand von dem Vorsprung (33) - den Anschlagbereich (36) bildet, wobei jeder Rückhalt (35) darüber hinaus eine Öffnung (37) aufweist, welche das Durchführen der zu verschiebenden Lasche (17) durch diese hindurch ermöglicht.

9. Vorrichtung (1) nach einem der Ansprüche 1 bis 7, **dadurch gekennzeichnet, dass** die Hülle (6) zwei Wände (25, 26) aufweist, welche zwischen einander einen Raum (27) begrenzen, in welchen der Stößel (5) verschiebbar eingreifen soll, wobei der Stößel (5) eine Innenwand (70) aufweist, in welcher jeweils der Anschlagbereich (36) vorgesehen ist, und eine äußere Wand (71), welche jeweils den Vorsprung (33) umfasst, wobei die Außenwand (71) sich jeweils über den Vorsprung (33) hinweg erstreckt und in den Raum (27) eingreift, wobei die Innen- (70) und die Außenwand (71) des Stößels zwischen sich jeweils eine Öffnung (37) begrenzen, und wobei die Lasche (17) jeweils in der Innenwand (25) der Hülle (6) vorgesehen ist.

10. Vorrichtung (1) nach einem der Ansprüche 1 bis 9, **dadurch gekennzeichnet, dass** das Behältnis durch einen Spritzenkörper (2) gebildet ist, welcher einen proximalen Flansch (10) und eine distale Abflussleitung aufweist, wobei der Spritzenkörper (2) ein Endstück zum Versprühen (3) aufweist, welches auf dessen distalem Ende aufgesteckt ist, welches einen Sprühkopf (11) bildet, welcher das Versprühen des Produkts, das in dem Spritzenkörper (2) enthalten ist, ermöglicht.

11. Vorrichtung (1) nach einem der Ansprüche 1 bis 9, **dadurch gekennzeichnet, dass** sie ein Behältnis (50) mit einem proximalen Boden (51) aufweist, ferner einen Kolben (53) mit einem mittigen, sich verjüngenden Bereich, eine Hohlnadel (54) zum Durchstechen dieses mittigen, sich verjüngenden Bereichs, und ferner einen Schaft (55), der in der Hülle (6) gehalten wird, wobei dieser Schaft (55) die Nadel (54) umfasst und eine Abflussleitung (56) für das Produkt bildet.

12. Vorrichtung (1) nach einem der Ansprüche 1 bis 9, **dadurch gekennzeichnet, dass** sie ein Behältnis (50) mit einem proximalen Boden (51) aufweist, ferner einen Kolben (60), welcher eine axiale Aussparung (61) aufweist, die in die distale Fläche des Kolbens (60) einmündet und die auf einer Seite des Kolbens (60) vorgesehen ist, wobei diese Aussparung (61) auf diese Weise einen Teil einer Seitenwand (60a) dieses Kolbens (60) umgrenzt, welcher in radiale Richtung des Kolbens biegsam ist, und einen Schaft (55), der in der Hülle (6) gehalten wird, welcher eine Abflussleitung (56) für das Produkt bildet, wobei dieser Schaft (55) dazu geeignet ist, gegen den Kolben (60) zu drücken, und zwar im Verlauf der Verschiebung des Stößels (5) im Verhältnis zur Hülle (6).

13. Vorrichtung (1) zum Versprühen oder Injizieren eines Produkts von Interesse in flüssiger Form, mit welcher zumindest eine vorbestimmte erste Dosis und eine vorbestimmte zweite Dosis des Produkts schrittweise abgegeben werden kann, wobei die Vorrichtung sich entlang einer Referenzachse von einem distalen Ende bis zu einem proximalen Ende erstreckt, und wobei sie Folgendes aufweist:
- ein sich axial erstreckendes Behältnis (2, 50), welches das Produkt enthält,
- einen Kolben (4, 53, 60), welcher in dem Behältnis vorliegt und welcher Letzteres verschließen kann, wobei der Kolben im Verhältnis zum Behältnis in Referenzrichtung axial verschiebbar ist, wodurch das Produkt distal aus dem Behältnis ausgetrieben werden kann,
- eine Hülle (6), welche zur Aufnahme und zur axialen Fixierung des Behältnisses angeordnet ist,
- einen mit einer Hülle (6) zusammengesetzten Stößel (5), der verschiebbar im Verhältnis zu Letzterem ist, und welcher derart angeordnet ist, dass er in axialen und proximalen Anschlag gegen das Behältnis oder den Kolben kommt, und dass er den Kolben in die Referenzrichtung verschiebt,
- Mittel (17, 33, 36, 37) zur Steuerung des Verschiebungsverlaufes des Stößels im Verhältnis zur Hülle, welche Mittel zur Aufteilung dieser Letzteren in einen ersten und einen zweiten Verlaufsteil angeordnet sind, durch welche die jeweilige Abgabe der ersten und zweiten Dosen bestimmt wird, wobei die Vorrichtung durch eine Hülle (6) und einen Stößel (5) charakterisiert ist, welche derart angeordnet sind, dass sie axial im Verhältnis zueinander in Richtung des Schubes verschiebbar sind, wodurch der Kolben in die Referenzrichtung verschoben wird, und
**dadurch**, dass die Mittel zur Steuerung Folgendes aufweisen:
- zumindest eine Lasche (17), die an dem Stößel (5) oder der Hülle (6) angebracht ist, und die zwischen einer ersten normalen, unbelasteten Position, in welcher die Lasche die axiale Verschiebung des Stößels nicht blockieren kann, und einer zweiten gebogenen erzwungenen Position beweglich ist, in welcher die Lasche die axiale Verschiebung des Stößels blockiert, wobei die Lasche an ihrem freien Ende einen Auflagebereich oder ein Auflageelement (17a) aufweist, welches dazu vorgesehen ist, gleichzeitig für den Stopp der axialen Verschiebung des Stößels (5) und für die Biegung der Lasche (17) zu sorgen, mit dem Effekt, dass der Stößel axial verschoben wird,
- zumindest eine Schräge (86), welche mit der Lasche von einem Anfangsende (86a) bis zu einem Schlussende (86b) zusammenwirkt, und welche jeweils an der Hülle (6) oder dem Stößel (5) angebracht ist, und gegen welche der Auflagebereich oder das Auflageelement (17a) der Lasche (17) aufsitzt, und zwar in Richtung des Schubes des Stößels (5), wobei die Schräge dazu vorgesehen ist, die Lasche von deren ersten normalen Position in deren zweite gebogene Position zu führen,
- zumindest einen Anschlag (36), der mit dem Auflagebereich oder dem Auflageelement (17a) der Lasche (17) zusammenwirkt, und der jeweils auf der Hülle (6) oder dem Stößel (5) angebracht ist, und zwar in Schubrichtung jeweils über oder unter das Schlussende (86b) der Schräge (86) hinweg, gegen welche der Auflagebereich oder das Auflageelement (17a) der Lasche (17) schließlich in deren zweiten gebogenen Position in Kontakt kommt,
- zumindest einen nicht aufliegenden Bereich oder Öffnung (22), welcher - wenn er auf der Lasche (17) vorgesehen ist - unter oder über dem Auflagenbereich oder das Auflagenelement (17a) hinweg angebracht ist, je nachdem die Lasche auf dem Stößel (5) oder der Hülle (6) vorgesehen ist, oder welcher,
- wenn er an der Hülle (6) vorgesehen ist - unter dem Anschlag (36) vorgesehen ist, wenn die Lasche (17) an dem Stößel (5) vorgesehen ist und das Auflageelement (17a) mit einer versenkten Schräge (86) zusammenwirkt, welche an der Hülle angebracht ist, wobei der nicht aufliegende Bereich oder Öffnung (22) dazu vorgesehen ist, den Rückhalt des Auflagenbereichs oder des Auflagenelements (17a) zu ermöglichen, und zwar bis zu dessen normalen, unbelasteten Position, ausgehend von der festgesetzten und gebogenen Position der Lasche (17), wenn der Schub auf den Stößel (5) nachlässt,
- einen ersten Verlaufsteil des Stößels (5), welcher für die Verschiebung des Letzteren bis zu dessen festgestellten axialen Position bestimmt ist, gefolgt von dem Kontakt des Auflagenbereichs oder des Auflagenelements (17a) mit dem Anschlag (36), und einem zweiten Verlaufsteil für die Verschiebung des Stößels über die axiale Position hinweg, in welcher der nicht aufliegende Bereich oder Öffnung (22) den Auflagenbereich oder das Auflagenelement (17a) aufgenommen hat.

14. Vorrichtung nach Anspruch 13, **dadurch gekennzeichnet, dass** die zweite gebogene Position der Lasche durch einen radialen Druck erzeugt wird.

15. Vorrichtung nach Anspruch 14, **dadurch gekennzeichnet, dass** die Lasche (17), welche an dem Stößel (5) oder der Hülle (6) vorgesehen ist, ein nicht aufliegendes Fenster (22) aufweist, welches im Verhältnis zum Auflagebereich oder dem Auflageelement (17a) zurücktritt; dass ferner die Schräge (86) zu einem Vorsprung (33) gehört, der jeweils an der Hülle (6) oder dem Stößel (5) vorgesehen ist, und dazu ausgebildet ist, durch das Fenster (22) frei hindurch zu dringen; dass ferner der Anschlag (36) derart eingerichtet ist, dass - in der zweiten gebogenen Position der Lasche - der Vorsprung (33) gegenüber dem Fenster (22) der Lasche zu liegen kommt, und danach mitten durch das Fenster dringt, wenn der Schub auf den Stößel (5) freigegeben wird, was den Rückzug der Lasche in deren erste Position bewirkt.

16. Vorrichtung nach Anspruch 13, **dadurch gekennzeichnet, dass** die zweite gebogene Position der Lasche durch tangentialen Druck erzeugt wird.

17. Vorrichtung nach Anspruch 16, **dadurch gekennzeichnet, dass** die Lasche (17) an dem Stößel (5) vorgesehen ist, der an seinem freiem Ende eine Ansatzkappe (17a) mit Querausdehnung gegen die Hülle (6) aufweist, eine vordere Fuge (84, 85) an der Lasche, welche deren tangentialen Spielraum von deren ersten normalen Position in deren zweite gebogene Position ermöglicht, dass ferner eine versenkte Schräge (86) an der Hülle (6) zur Aufnahme des Stößels (17a) vorgesehen ist, die sich vom Anfangsende (86a) bis zum Schlussende (86b) merklich schrägwinklig in der Wand der Hülle erstreckt; dass der Anschlag, der mit der Ansatzkappe (17a) zusammenarbeitet, an der Hülle über das Schlussende (86b) der Schräge (86) hinweg vorgesehen ist und welcher für eine winklige, aber kontinuierliche Verbindung der Schräge (86) mit einem Rückhalt der Schräge (90) bestimmt ist; dass der nicht aufliegende Bereich oder Öffnung (22), welcher an der Hülle (6) unter dem Anschlag (36) versenkt angebracht ist, zur Aufnahme der Ansatzkappe (17a) angepasst ist, wenn die Lasche (17) in ihre normale Position zurückkehrt; und dass sich ein axialer Schlitz (91) kontinuierlich über den nicht aufliegenden Bereich über den Letzteren hinweg erstreckt.

18. Vorrichtung nach Anspruch 17, **dadurch gekennzeichnet, dass** der axiale Schlitz (91) in eine durchgehende Öffnung (92) der Hülle (6) mündet, wobei der zweite Verlaufsteil des Stößels (5) zum Anschlag des Kolbens (4) gegen die Wand des Behältnisses (2) bestimmt ist.

19. Vorrichtung nach Anspruch 17, **dadurch gekennzeichnet, dass** unter dem Anfangsende (86a) der Schräge (86) - und kontinuierlich mit der Letzteren - eine Halteaufnahme für die Ansatzkappe (17a) vorgesehen ist, insbesondere um eine endgültige Verbindung des Stößels (5) und der Hülle (6) zu ermöglichen.

20. Vorrichtung nach Anspruch 17, **dadurch gekennzeichnet, dass** über den nicht aufliegenden Bereich oder Öffnung (22) hinaus - und kontinuierlich mit Letzterem - eine Halteaufnahme (94) für die Ansatzkappe (17a) vorgesehen ist.

21. Vorrichtung nach Anspruch 13, **dadurch gekennzeichnet, dass** der eine Teil zumindest eine Zunge (87) aufweist, welche getrennt oder unabhängig von der Lasche (17) ist, und welche an dem Stößel (5) oder der Hülle (6) angebracht ist, wonach die Lasche (17) an dem Stößel (5) oder der Hülle (6) angebracht ist, und zwar beweglich zwischen einer ersten normalen, nicht belasteten Position, in welcher die Zunge die axiale Verschiebung des Stößels (5) nicht blockiert, und einer zweiten gebogenen, belasteten Position, in welcher die Zunge (87) zu der Rückstellung des Stößels (5) beiträgt, um die Rückführung der Lasche (17), ausgehend von deren festgestellten und gebogenen Position, in deren normale Position zu ermöglichen, und dass der andere Teil, nämlich die Schräge (86) und das freie Ende (87a) der Zunge (87) zur Zusammenwirkung des einen mit dem anderen ausgebildet sind, und zwar derart, dass - während des ersten Teilverlaufs des Stößels - sich die Zunge im gebogenen Zustand befindet, und dass die Zunge (87) - während des zweiten Teilverlaufs des Stößels - von der Schräge freikommt und in ihre normale Position zurückkehrt.

22. Vorrichtung nach Anspruch 21, **dadurch gekennzeichnet, dass** die zweite gebogene Position der Zunge (87) durch radialen Druck erzeugt wird, wenn die zweite gebogene Position der Lasche (17) durch radialen Druck erzeugt wird.

23. Vorrichtung nach Anspruch 21, **dadurch gekennzeichnet, dass** die zweite gebogene Position der Zunge (87) durch tangentialen Druck erzeugt wird, wenn die zweite gebogene Position der Lasche (17) durch tangentialen Druck erzeugt wird.

24. Vorrichtung nach Anspruch 22, **dadurch gekennzeichnet, dass** die Lasche (17), die an dem Stößel (5) oder der Hülle (6) angebracht ist, ein nicht aufliegendes Fenster (22) umfasst, dass die Schräge (86) zu einem Vorsprung (33) gehört, welcher jeweils an der Hülle (6) oder dem Stößel (5) angebracht ist, und zum freien Durchdringen des Fensters (22) angepasst ist; ferner, dass der Anschlag (36) derart ausgebildet ist, dass - in der zweiten gebogenen Position der Lasche - der Vorsprung (33) gegenüber dem Fenster (22) der Lasche zu liegen kommt und dann mittendurch das Fenster hindurchreicht, wenn der Schub des Stößels (5) gelockert wird, was den Rückzug der Lasche in ihre erste Position bewirkt.

25. Vorrichtung nach Anspruch 24, **dadurch gekennzeichnet, dass** die Zunge (87) in der Mitte des Fensters (22) vorgesehen ist, dass das freie Ende (87a) der Zunge (87) sich unter dem Kopf (17a) befindet und zwei Ohren (88) zum Aufliegen gegen die Schräge (86) aufweist, und andererseits die Schräge (90) zwei Flügel (331) und (332) aufweist, die zu beiden Seiten eines Schlitzes (89) angebracht sind, um den freien Übertragungsdurchgang der Zunge zu ermöglichen, wobei jedes Ohr (88) in Schubrichtung des Stößels (5) mit einem Teil der Schräge (86) in Kontakt kommt, welcher als Flügel (331, 332) definiert ist, um über den ersten Verlaufsteil des Stößels (5) hinweg aus dem Teil der Schräge hinauszugelangen.

## Claims

1. A spray or injection device (1) making it possible to deliver at least two preset doses of product, comprising:
- a container (2, 50) containing the product to be sprayed or to be injected,
- a plunger (4, 53, 60) placed in this container (2, 50) which is able to move in this container (2, 50) in order to extract the product from the container (2, 50),
- a casing (6) in which the container (2, 50) is placed,
- a pusher (5) which can be moved with respect to the casing (6) to allow the relative movement of the container (2, 50) and of the plunger (4, 53, 60), and
- means (17, 33, 36, 37) making it possible to subdivide the length of travel of the pusher (5) with respect to the casing (6) into at least two travel portions, each one corresponding to the delivery of a dose of product;
the device (1) being **characterized in that**:
- the casing (6) or the pusher (5) comprises at least one tab (17) which can be moved radially between a first normal radial position, in which the tab (17) provides no obstacle to the movement of the pusher (5) with respect to the casing (6), and a second radial position, in which the tab (17) provides an obstacle to this movement, this tab (17) comprising a pressing region (17a) and a nonpressing region (22);
- the pusher (5) or the casing (6), respectively, comprises at least one ramp-shaped projection (33), against which said pressing region (17a) of the tab (17) comes to bear during the movement of the pusher (5) with respect to the casing (6) in the direction allowing the product to be sprayed or injected, which brings said tab (17) into said second radial position, then opposite which comes said nonpressing region (22) of the tab (17), which allows said tab (17) to return to said first radial position; the pusher (5) or the casing (6), respectively, further comprises at least one stop region (36) against which the tab (17) presses when it is brought into said second radial position by said projection (33), this pressure occurring just before said projection (33) comes opposite said nonpressing region (22),
the device (1) being designed such that the release of the force exerted on the pusher (5), which allows this pusher (5) to move with respect to the casing (6), makes it possible to free the pressure of the tab (17) against said stop region (36) and therefore allows said tab (17) to return to said first radial position, said projection (33) then coming opposite said nonpressing region (22), this return of said tab (17) to said first radial position making it possible to free the movement of the pusher (5) for a following portion of length of travel, for the purpose of spraying or injecting a following dose of the product.

2. The device (1) as claimed in claim 1, **characterized in that** said nonpressing region of the tab (17) is formed by a window (22) made in the tab (17) set back from the end of this tab, said pressing region being formed by the region (17a) of this tab connecting this end of the tab and this window (22).

3. The device (1) as claimed in claim 1 or claim 2, **characterized in that** it comprises at least two tabs (17), two projections (33) and two stop regions (36), which are diametrically opposed.

4. The device (1) as claimed in one of claims 1 to 3, **characterized in that** the casing (6) and the pusher (5) comprise means (20, 21, 38) making it possible to form at least one "hard point" having to be crossed at the start of delivering a dose.

5. The device (1) as claimed in claim 4, **characterized in that** said means making it possible to form at least one "hard point" consist of at least one lug (20, 21) projecting laterally from a tab (17) as mentioned above and of at least one boss (38) made in a suitable corresponding location of the casing (6).

6. The device (1) as claimed in one of claims 1 to 5, **characterized in that** the whole pusher (5) is made from a slightly flexible common plastic, the mobility of each tab 17 resulting from the flexibility of this material, no tab (17) being deformed in said first radial position and each tab (17) being elastically deformed in said second radial position.

7. The device (1) as claimed in one of claims 1 to 6, **characterized in that** each tab (17) has a distal head (17a) with projecting edges, forming, with a complementary means comprised in the casing (6), snap-fastening means making it possible to prevent the separation of the pusher (5) from the casing (6) after the engagement of the pusher in the casing.

8. The device (1) as claimed in one of claims 1 to 7, **characterized in that** the casing (6) comprises two walls (25, 26) defining between them a space (27) in which the pusher (5) is designed to be engaged so that it can slide, each projection (33) which projects from the outer face of the inner wall (25) and the outer wall (26) comprising at least one return (35) forming, inside said space (27) and at a preset distance from the projection (33), said stop region (36), each return (35) further comprising an opening (37) which allows the tab (17) to pass therethrough by sliding.

9. The device (1) as claimed in one of claims 1 to 7, **characterized in that** the casing (6) comprises two walls (25, 26) defining between them a space (27) in which the pusher (5) is designed to be engaged so that it can slide, the pusher (5) comprising an inner wall (70), in which each stop region (36) is made, and an outer wall (71) comprising each projection (33), this outer wall (71) extending beyond each projection (33) and being engaged in said space (27), said inner (70) and outer (71) walls of the pusher (5) defining each opening (37) between them; each tab (17) is made in said inner wall (25) of the casing (6).

10. The device (1) as claimed in one of claims 1 to 9, **characterized in that** the container is formed by a syringe body (2) comprising a proximal collar (10) and a distal flow conduit, this syringe body (2) comprising a spray nozzle (3) fitted onto its distal end, which forms a spray head (11) making it possible to spray the product contained in the syringe body (2).

11. The device (1) as claimed in one of claims 1 to 9, **characterized in that** it comprises a container (50) with a proximal bottom (51), a plunger (53) having a thinned central region, a hollow needle (54) suitable for piercing this thinned central region, and a rod (55) held on the casing (6), this rod (55) comprising the needle (54) and forming a flow conduit (56) for the product.

12. The device (1) as claimed in one of claims 1 to 9, **characterized in that** it comprises a container (50) with a proximal bottom (51), a plunger (60) comprising an axial recess (61) which opens out into the distal face of the plunger (60) and which is made on one side of this plunger (60), this recess (61) thus defining a side wall portion (60a) of this plunger (60) having flexibility in the radial direction of the plunger, and a rod (55) held on the casing (6), forming a flow conduit (56) for the product, this rod (55) being suitable for pressing against the plunger (60) during movement of the pusher (5) with respect to the casing (6).

13. A spray or injection device (1) for spraying or injecting a product of interest, in liquid form, making it possible to deliver at least a first and a second preset dose of said product in succession, said device extending along a reference axis from a distal end to a proximal end, comprising:
- an axially elongate container (2, 50) containing said product,
- a plunger (4, 53, 60) placed in the container and blocking off the latter, said piston being axially moveable with respect to the container, in a reference direction, allowing said product to be propelled distally from the container,
- a casing (6) designed to accommodate and axially secure said container,
- a pusher (5) assembled with the casing (6), moveable with respect to the latter, designed to come into axial and proximal abutment against the container or the plunger, and to move said plunger in the reference direction,
- means (17, 33, 36, 37) for controlling the length of travel of the pusher with respect to the casing, these means being designed to divide this travel into a first travel portion and a second travel portion, determining the respective delivery of the first and second doses, said device being **characterized in that** the casing (6) and the pusher (5) are designed to be axially moveable one with respect to the other in a pushing direction, generating the movement of the plunger in the reference direction, and **in that** the control means comprise:
- at least one tab (17) arranged on the pusher (5) or the casing (6), able to move between a first, unstressed, normal position in which said tab does not block the axial movement of the pusher, and a stressed, flexed, second position in which said tab halts the axial movement of the pusher, said tab at its free end comprising a pressing region or element (17a) designed to contribute both to the halting of the axial movement of the pusher (5) and to the flexing of the tab (17) under the effect of the axial movement of the pusher,
- at least one ramp (86) cooperating with said tab from an end known as the initial end (86a) to an end known as the final end (86b) and arranged respectively on the casing (6) or on the pusher (5), against which the pressing region or element (17a) of said tab (17) bears in the direction of pressure of said pusher (5), said ramp being designed to bring said tab from its normal first position to its flexed second position,
- at least one stop (36) cooperating with the pressing region or element (17a) of the tab (17), arranged respectively on the casing (6) or the pusher (5), respectively beyond or before the final end (86b) of the ramp (86) in the direction of pressure, against which the pressing region or element (17a) of the tab (17) finally abuts in its flexed second position,
- at least one nonpressing region or opening (22) which is either arranged on the tab (17) before or beyond said pressing region or element (17a), depending on whether said tab is arranged on the pusher (5) or on the casing (6), or arranged on the casing (6), before the stop (36) when the tab (17) is arranged on the pusher (5) and the pressing element (17a) cooperates with a hollowed ramp (86) formed on the casing, said nonpressing region or opening (22) being designed to allow said pressing region or element (17a) to return to its unstressed normal position from the halted and flexed position of the tab 17 when the pressure on the pusher (5) is released,
- the first portion of travel of the pusher (5) being determined by the movement of this pusher as far as its axial stop position, following contact between the pressing region or element (17a) and the stop (36), and the second portion of travel being determined by the movement of the pusher beyond the axial position in which the nonpressing region or opening (22) has accommodated the pressing region or element (17a).

14. The device as claimed in claim 13, **characterized in that** the flexed second position of the tab is obtained by radial stress.

15. The device as claimed in claim 14, **characterized in that** the tab (17) arranged on the pusher (5) or the casing (6) comprises a nonpressing window (22) set back with respect to said pressing region or element (17a); the ramp (86) belongs to a projection (33) arranged respectively on the casing (6) or the pusher (5) and designed to pass freely through said window (22); the stop (36) is arranged such that, in the flexed second position of said tab, the projection (33) faces the window (22) of the tab, then penetrates through said window when the pressure on the pusher (5) is released, thus causing said tab to return to its first position.

16. The device as claimed in claim 13, **characterized in that** the flexed second position of the tab is obtained by tangential stress.

17. The device as claimed in claim 16, **characterized in that** the tab (17) is arranged on the pusher (5), at its free end comprises a stud (17a) extending transversely toward the casing (6), an anterior cut (84, 85) to said tab allowing it to deflect tangentially from its first, normal, position to its second, flexed, position, the ramp (86) is arranged on the casing (6), designed with a hollow to accommodate the stud (17a), and extends more or less obliquely in the wall of the casing, from the initial end (86a) to the final end (86b); the stop (36), cooperating with the stud (17a), arranged on the casing (6) beyond the final end (86b) of the ramp (86) is determined by the joint, at an angle, but with continuity, of said ramp (86) with a ramp return (90); the nonprossing region or opening (22), arranged in a hollow on the casing (6) before the stop (36) is designed to accommodate the stud (17a) when the tab (17) returns to its normal position; and an axial slot (91) extends, continuous with the nonpressing region (22), beyond the latter.

18. The device as claimed in claim 17, **characterized in that** the axial slot (91) opens out freely into a through opening (92) in the casing (6), the second portion of travel of the pusher (5) being determined by the abutment of the plunger (4) against the wall of the container (2).

19. The device as claimed in claim 17, **characterized in that**, before the initial end (86a) of the ramp (86), and continuous with the latter, there is formed a housing (93) in which to park the stud (17a), particularly to allow definitive assembly of the pusher (5) and of the casing (6).

20. The device as claimed in claim 17, **characterized in that**, beyond the nonpressing region or opening (22), and continuous with the latter, there is formed a housing (94) in which to park the stud (17a).

21. The device as claimed in claim 13, **characterized in that**, on the one hand, it comprises at least one tongue (87) distinct from or independent of the tab (17), arranged on the pusher (5) or the casing (6) depending on whether the tab (17) is arranged on the pusher (5) or on the casing (6), able to move between an unstressed normal first position in which said tongue does not impede the axial movement of the pusher (5) and a stressed flexed second position in which said tongue (87) contributes to returning the pusher (5) to allow said tab (17) to return to its normal position, from its flexed and halted position, and, on the other hand, the ramp (86) and the free end (87a) of the tongue (87) are designed to cooperate with one another so that, during the first portion of travel of the pusher, the tongue is flexed and during the second portion of travel of the pusher, the tongue (87) escapes from the ramp and returns to its normal position.

22. The device as claimed in claim 21, **characterized in that** the flexed second position of the tongue (87) is obtained by radial stress when the flexed second position of the tab (17) is obtained by radial stress.

23. The device as claimed in claim 21, **characterized in that** the flexed second position of the tongue (87) is obtained by tangential stress when the flexed second position of the tab (17) is obtained by tangential stress.

24. The device as claimed in claim 22, **characterized in that** the tab (17) arranged on the pusher (5) or the casing (6) comprises a nonpressing window (22), the ramp (86) belongs to a projection (33) arranged respectively on the casing (6) or on the pusher (5) and designed to pass freely through said window (22); the stop (36) is arranged in such a way that, when said tab is in the flexed second position, the projection (33) faces the window (22) of the tab then penetrates through said window when the pressure on the pusher (5) is released, thus causing said tab to return to its first position.

25. The device as claimed in claim 24, **characterized in that**, on the one hand, the tongue (87) is arranged at the center of the window (22), the free end (87a) of the tongue (87) remaining before the head (17a) and comprising two ears (88) for pressing against the ramp (86) and, on the other hand, the ramp (90) comprises two flanges (331) and (332) arranged one on each side of a slot (89) allowing the tongue free passage in translation, each ear (88) coming into contact with a ramp portion (86) defined by a flange (331, 332) in the direction of pressure of the plunger (5) so as to escape from said ramp portion beyond the first portion of travel of the pusher (5).
